(19) Europäisches Patentamt

European Patent Office

Office européen des brevets

(11) **EP 1 112 992 B1**

(12) **FASCICULE DE BREVET EUROPEEN**

(45) Date de publication et mention
de la délivrance du brevet:
**06.04.2005 Bulletin 2005/14**

(51) Int Cl.⁷: **C07C 31/08**

(21) Numéro de dépôt: **01810003.2**

(22) Date de dépôt: **03.01.2001**

(54) **Procédé pour la préparation des mélanges alcools-eau**

Verfahren zur Herstellung von Alkohol-Wasser-Gemischen

Process for the preparation of alcohol/water mixtures

(84) Etats contractants désignés:
**AT BE CH CY DE DK ES FI FR GB GR IE IT LI LU
MC NL PT SE TR**

(30) Priorité: **03.01.2000 CH 62000**

(43) Date de publication de la demande:
**04.07.2001 Bulletin 2001/27**

(73) Titulaire: **Liebich, Bernard W., Dr.
3072 Ostermundigen (CH)**

(72) Inventeur: **Liebich, Bernard W., Dr.
3072 Ostermundigen (CH)**

(74) Mandataire: **Fischer, Franz Joseph
IPTO S.A.,
P.O. Box 594
3000 Bern 25 (CH)**

(56) Documents cités:
- **B.W. LIEBICH: "Etude approfondie de la
  contraction dans les mélanges alcools-eau.
  Partie 1: calculs, représentations et nouveaux
  résultats sur la signification de la contraction
  dans les mélanges éthanol-eau" MITTEILUNGEN
  AUS DER LEBENSMITTELUNTERSUCHUNG
  UND HYGIENE, vol. 90, no. 6, décembre 1999
  (1999-12), pages 714-733, XP008008704
  Bundesamt für Gesundheitswesen, Berne, CH
  ISSN: 1424-1307**
- **CHEMICAL ABSTRACTS, vol. 96, no. 16, 19 avril
  1982 (1982-04-19) Columbus, Ohio, US; abstract
  no. 129625u, M. LATTARULO, ET AL.:
  "Water-alcohol mixtures" page 449;
  XP002214704 -& M. LATTARULO, ET AL.:
  "Miscele acqua-alcool" BOLLETINO DELLA
  SOCIETA ITALIANA DI FARMACIA
  OSPEDALIERA, vol. 27, no. 5, 1981, pages
  319-322, XP008008663 Edizione Minerva Medica,
  Torino, IT**
- **B.W. LIEBICH: "Etude approfondie de la
  contraction dans les mélanges alcools-eau.
  Partie 2: nouvelle méthode pour la calcul, la
  représentation et l'application de la contraction
  partielle aux mélanges éthanol-eau"
  MITTEILUNGEN AUS
  LEBENSMITTELUNTERSUCHUNG UND
  HYGIENE, vol. 91, no. 6, décembre 2000
  (2000-12), pages 700-722, XP008008660
  Bundesamt für Gesundheitswesen, Berne, CH
  ISSN: 1424-1307**

**Description**

L' invention s'occupe de la préparation des mélanges alcools-eau très précis se basant sur une série de recherches sur la contraction totale et partielle lors de la dilution de l'alcool éthylique, spiritueux et alcools avec l'eau.

Après des études approfondies de la contraction de volume dans les mélanges éthanol-eau, connue en alcoométrie comme "contraction", l'inventeur a proposé des formules détaillées pour définir cet effet. Cet effet a été non seulement représenté par rapport aux % masse et % volume, mais aussi ce qui est nouveau, en fonction des % mol. Cette représentation de la contraction fait apparaître de manière nouvelle dans les mélanges éthanol-eau un maximum situé entre 20 et 25 % mol, se trouvant aux mêmes % mol que le maximum observé par exemple pour la viscosité et celui obtenu par d'autres mesures physico-chimiques (1-8). D'autre part une augmentation de la contraction lors de la diminution de la température est aussi clairement mise en évidence. Les variations d'intensité de la contraction en fonction des % mol sont en outre interprétés à l'aide de la méthode dite "des variations continues", grâce à laquelle la présence d'associations moléculaires entre les molécules d'alcools et d'eau, responsables de ces contractions de volumes pendant la dilution de mélanges alcool-eau, a aussi été confirmée (9-14).

Dans le cadre de cette étude on a ensuite été amené à étudier et développer la notion nouvelle des "contractions partielles". La connaissance détaillée de ces effets est en particulier indispensable pour pouvoir calculer avec précision la préparation des solutions alcooliques en passant aussi par les volumes.

On pourra ainsi non seulement trouver une teneur en alcool déterminée, mais aussi ce, qui est aussi nouveau, un volume final précis à une température donnée dans le cas par exemple des dilutions suivantes.

Ainsi lorsqu'on utilise un volume de mélange initial ou final déterminé, ou que l'on rajoute à un premier mélange alcool-eau d' un deuxième mélange alcool-eau, on pourra distinguer les méthodes de dilutions pratiques 2a et 2b selon cette description.

Le document Lattarulo, M., Bianco, P., Boll. Soc. Ital. Osp. 1981, 27(5), 319-322 décrit un procédé pour obtenir 100 ml d'un mélange alcool-eau avec un degré d'alcool désiré (pour l'utilisation dans la pharmaceutique et dans l'analytique). En considérant une contraction de volume partielle, le procédé se sert d'eau pure et de alcool à 95° (éthanol). On met à disposition une formule et un tableau pour la pratique. La publication est restreinte à un seul cas, notamment celui utilisant de l'eau et de l'alcool à 95° comme produits de départ. Dans ce document on ne donne pas d'indications pour la préparation de mélanges alcool-eau avec un degré désiré en se servant de deux mélanges alcool-eau de départ avec d'autres teneurs et en ne considérant pas l'interaction complexe des contractions partielles et totales.

Le but de la présente invention est donc de proposer des techniques pour la préparation de mélanges alcool-eau, avec des teneurs en alcool et des volumes bien déterminés, qui se basant sur les méthodes de dilution développées ici tiennent compte avec précision de la contraction. Ii est prévu d'adapter les données obtenues ici, à un procédé informatisé permettant le pilotage électronique d'un système de remplissage de mélanges alcools-eau. Un tel système pourra ainsi être d'une grande utilité par exemple pour l'industrie des boissons alcooliques, qui travaille avec un produit dont la charge fiscale est considérable.

Une des caractéristiques de l'alcoométrie vient du fait, que lors de l'addition de volumes déterminés d'alcool éthylique et d'eau, on obtient un volume final notablement inférieur à la somme des deux volumes ajoutés (17-21). La contraction, qui visiblement a été considérée comme accessoire en alcoométrie où elle est simplement désignée sous le nom de <u>contraction</u> (C), n'a à notre connaissance jamais été étudiée de manière approfondie, ce qui rendait jusqu'à maintenant certaines opérations de mélanges difficiles et plutôt approximatives. Le présent procédé basé sur une série de connaissances nouvelles sur la contraction et son effet structurel, comme aussi les interactions faibles entre les molécules d'alcool et d'eau, permettra à n'en pas douter d'améliorer cette situation.

La contraction est donc étudiée et calculée ici de manière nouvelle pour les mélanges éthanol-eau ($C_2H_5OH$-$H_2O$), aussi sous forme de nouvelles tables selon la présente description. Ce travail est facilité par d'excellentes valeurs des diverses tables alcoométriques officiellement utilisées en Suisse et sur le plan international (22-28), toutes établies conformément aux recommandations de l'Organisation Internationale de Métrologie Légale (OIML)

Il sera ensuite montré que la technique développée ici pour calculer la contraction dans les mélanges éthanol-eau est aussi valable de manière plus générale pour le calcul de la contraction de mélanges, tels par exemple ceux des alcools monohydroxylés avec l'eau.

On doit se baser dans ces cas sur d'autres tables donnant avec beaucoup moins de précision les % mas, les % vol et les masses volumiques ($kg/m^3$) pour ces autres substances spécifiques.

Il sera aussi examiné, quelle relation peut exister pour les mélanges alcool-eau entre les résultats observés pour la contraction et ceux obtenus pour d'autres effets et par diverses mesures physico-chimiques. Au sujet des mélanges alcool-eau, des études détaillées mettent montrent que les anomalies observées dans ces mélanges sont en générale en relation avec des questions de structure (1-8). Ainsi sur la base de différentes recherches physico-chimiques, comme par exemple par les techniques des ultrasons, la résonance nucléaire magnétique, l'anisotropie de la fluorescence, la viscosité etc. la formation d'associations moléculaires dans les mélanges éthanol-eau avec un maximum situé dans la région de 20 à 25 % mol a parfois été discutée. D'autre part il est aussi à noter que les diverses caractéristiques physico-chimiques observées pour les mélanges alcool-eau s'écartent fortement de celles trouvées d'un côté pour l'eau et de l'autre pour les alcools liquides purs.

L'objet de la présenté invention est ce procédé pour la préparation de mélanges alcool-eau, en mélangeant des mélanges alcool-eau afin d'obtenir ainsi de nouveaux mélanges avec des teneurs en alcool et des volumes très bien déterminés selon la définition dans la revendication 1. Ce procédé est basé sur les résultats des recherches décrites ci-dessous. Les cas spéciaux selon les méthodes 1a et 1 b sont indiqués pour mieux comprendre le principe de la présente invention.

Liste des figures

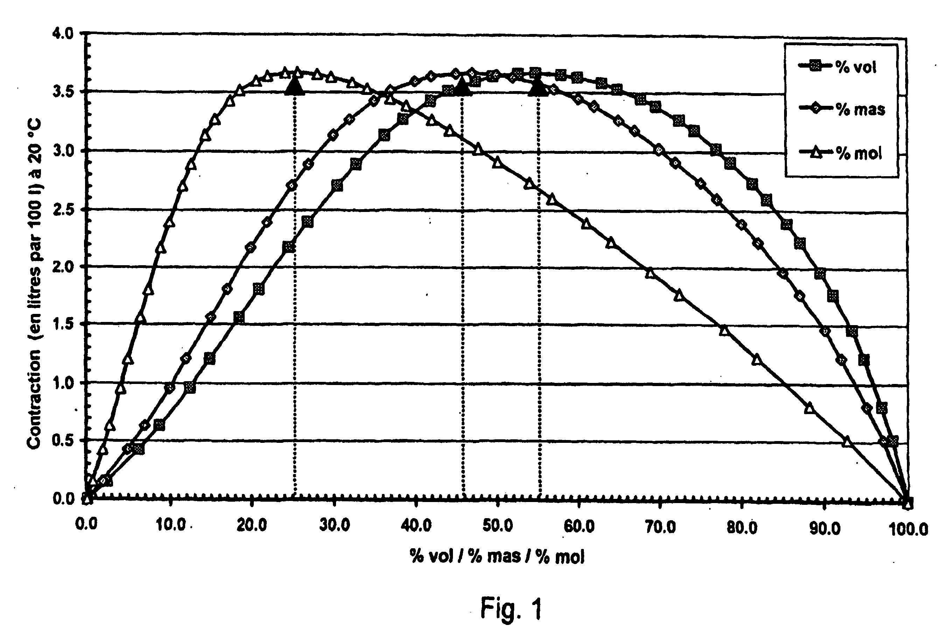

Fig. 1:    Courbes des contractions pour les mélanges éthanol-eau, en litres par 100 l, en fonction de % mas, % vol et en % mol à 20 °C.

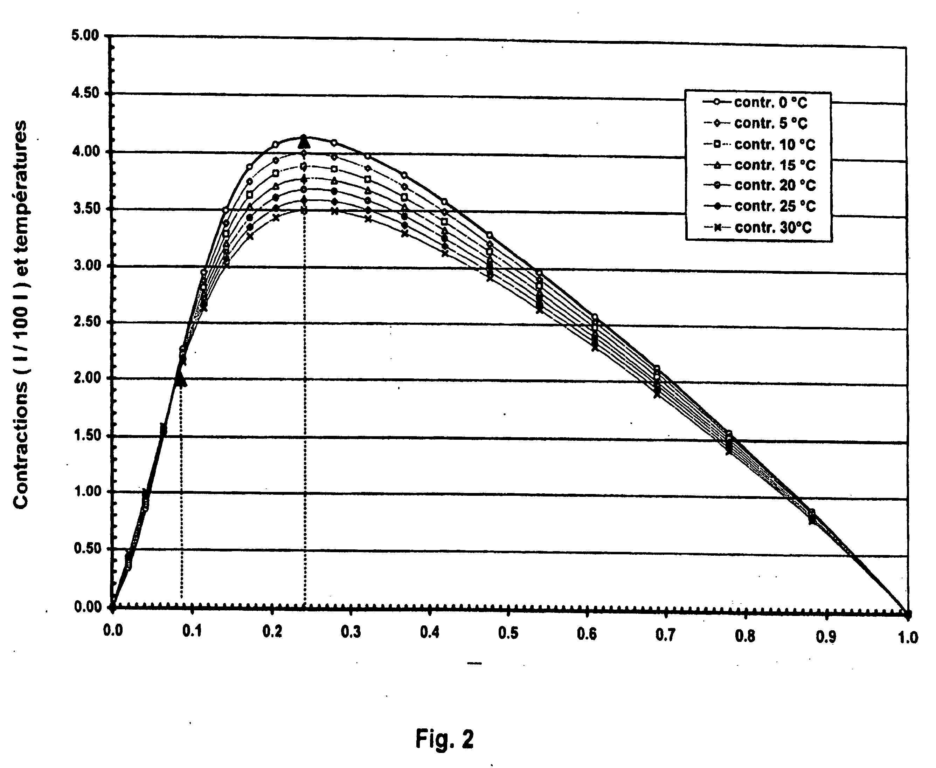

Fig. 2:    Courbes de contraction pour les mélanges éthanol-eau, en litres par 100 l, représentées en fonction des % mol de 0 à 30 °C, on peut observer qu'elles se ressemblent, mais augmentent avec la diminution de la température.

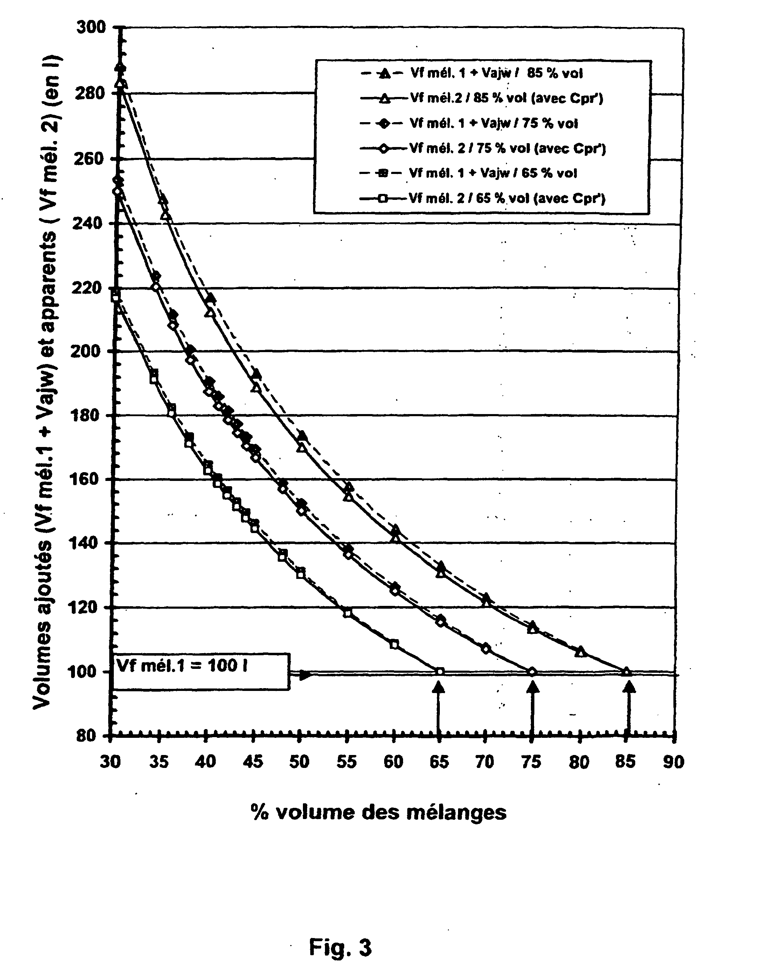

Fig. 3:    Diagramme de dilution selon la méthode 1a, par addition d'eau à 100 l de mélanges à 65, 75 et 85 % vol, montrant la différence entre les volumes finals apparents ($Vf_{mél\,2}$), qui tiennent compte des contractions partielles restantes ($C_{pr}'$) ( cf. ligne pleine), et ($V_{aj\,W}$) à ajouter à ($Vf_{mél.1}$), qui n'en tiennent pas compte (cf. ligne pointillée).

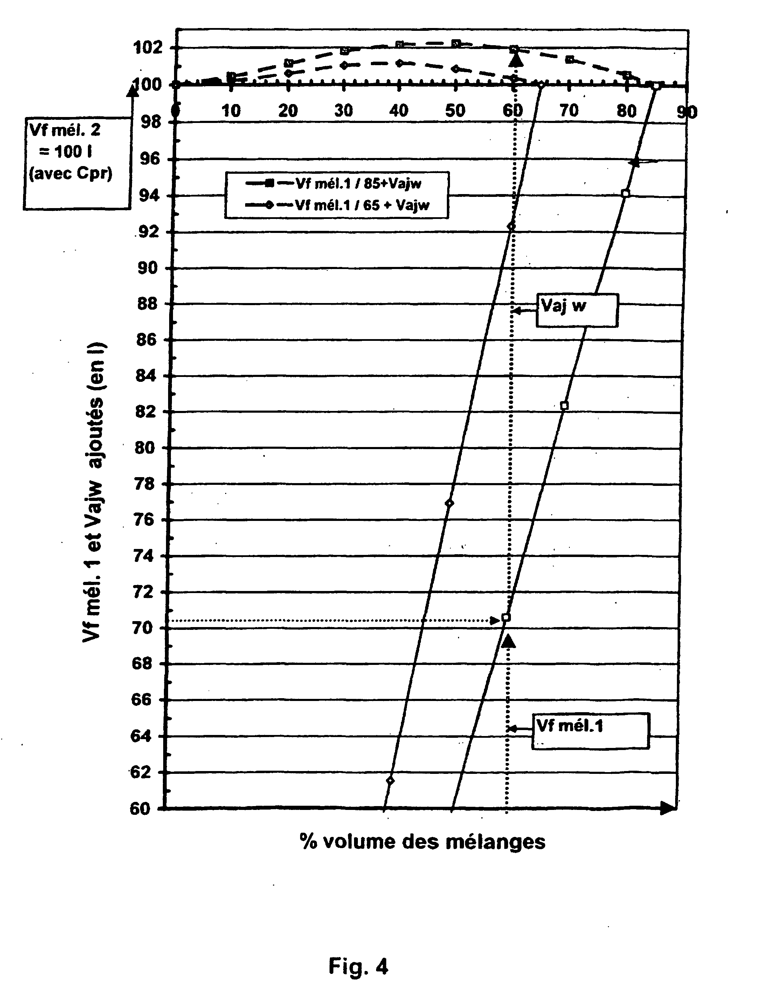

Fig. 4:    Diagramme de dilution selon la méthode 1b pour l'obtention d'un mélange avec un volume final apparent ($Vf_{mél.\,2}$) fixe de 100 l, où l'eau est ajoutée à des mélanges éthanol-eau de 65 et 85 %vol. La contraction partielle restante ($C_{pr}$) y est montrée, comme différence entre les volumes d'eau ajoutés ($V_{aj\,W}$) à ($Vf_{mél.\,1}$) (cf. ligne pointillée), et les volumes ($Vf_{mél.\,2}$) de 100 l (cf. ligne pleine).

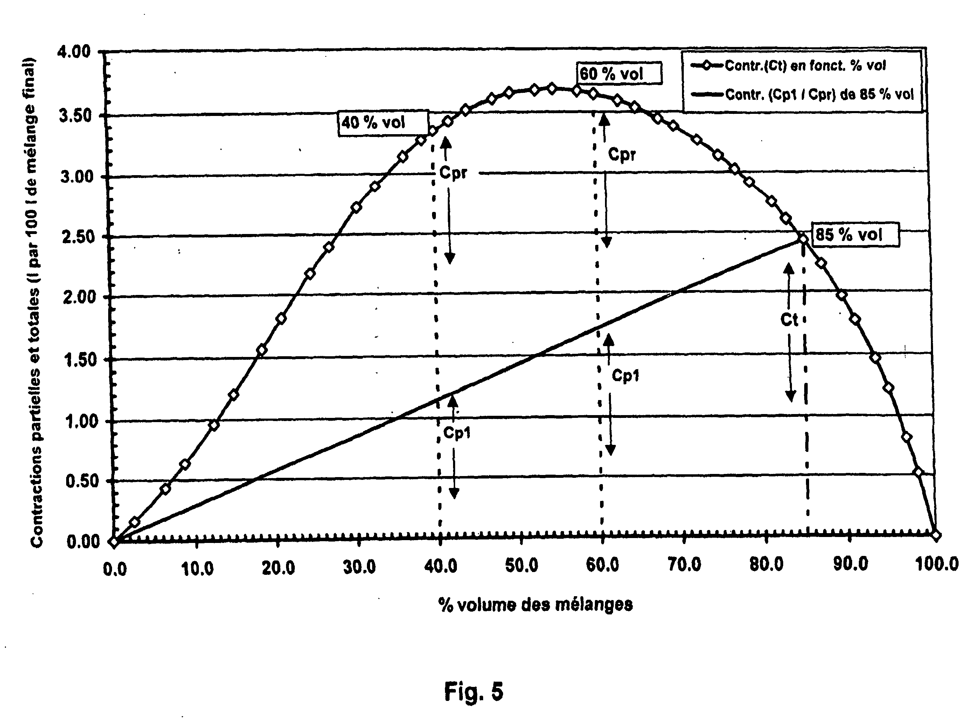

Fig. 5:    Diagramme des contractions en l par 100 l en fonction des % vol, mettant en évidence pour les méthodes 1a et 1b les contractions totales ($C_t$) des mélanges ($Vf_{mél.\,1}$) de 85 % vol et 65 % vol, et les contractions partielles ($C_{p1}$) et ($C_{pr}$) lors des dilutions pour obtenir un volume final apparent ($Vf_{mél.\,2}$) à 40 ou 60 % vol.

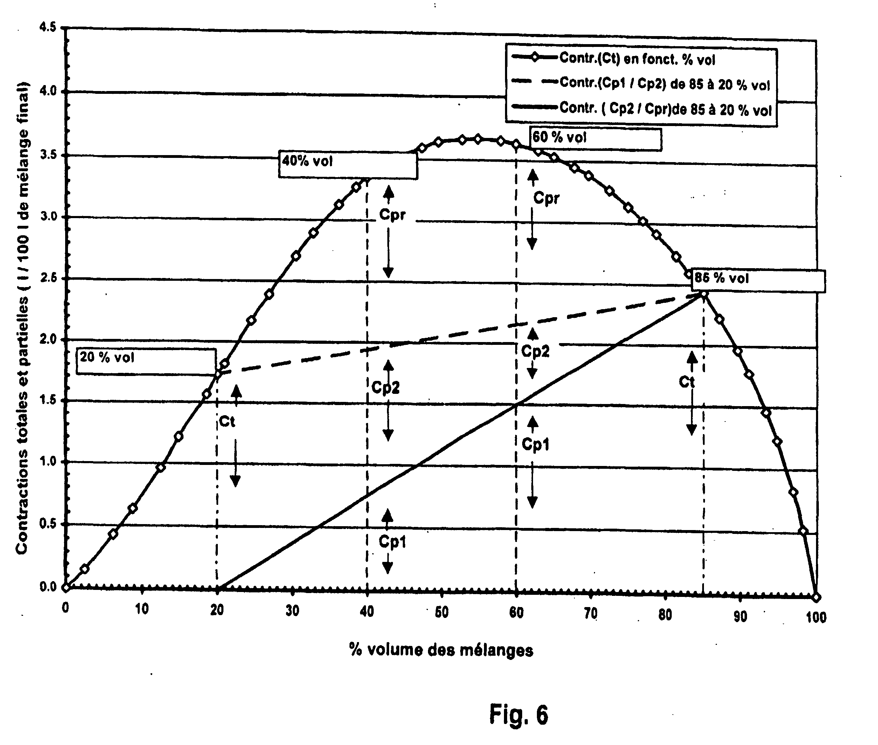

Fig. 6:    Diagramme des contractions en par 100 l, montrant la dilution selon les méthodes 2a et 2b de mélanges éthanol-eau avec d'autres mélanges éthanol-eau d'un % vol inférieure. La dilution d'un mélange de 85 % vol avec un autre de 20 % vol, pour obtenir un volume final apparent ($Vf_{mél\,2}$) de 40 ou 60 % vol, met en évidence avec les droites de dilution, les contractions partielles ($C_{p1}$), ($C_{p2}$) et ($C_{pr}$).

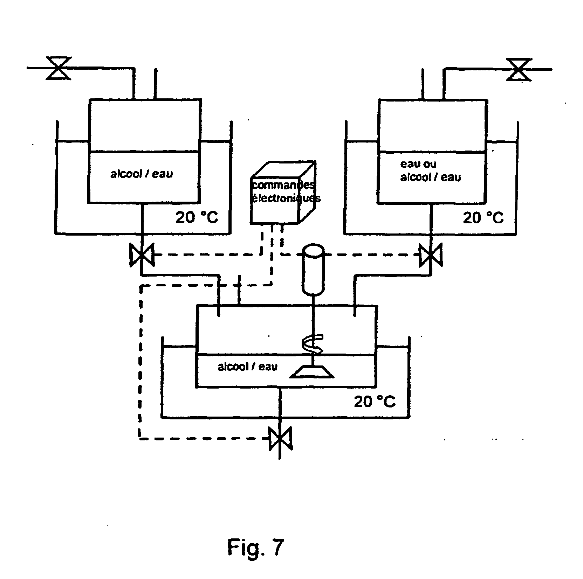

Fig. 7:    Schéma d'un dispositif à commandes électroniques, pour la préparation selon le procédé de l'invention de mélanges alcool-eau par addition d'autres mélanges alcool-eau ou d'eau , avec une teneur en % vol et un volume bien déterminés à la température de 20 °C.

**Définition et détermination de la contraction**

Les termes utilisés ici, pour la «contraction» ($C$), la «contraction totale» ($C_t$), et la «contraction partielle» ($C_p$), ainsi que la notion de normalisation seront donc définis et expliqués comme suit:

La contraction: Pour un volume final de mélange donné la contraction ($Vf_{mél.}$)* est égale à la différence entre les grandeurs a) et b) suivantes :

a) Le volume d'eau ou de mélange alcool-eau ajouté au mélange initial, qui correspond à la somme des volumes effectivement ajoutés, que nous appellerons ici <u>volume ajouté d'eau</u> ($V_{aj\,W}$)* ou <u>volume ajouté de mélange</u> ($V_{aj\,mél.}$)*.

b) Le volume apparent de l'eau ou du mélange alcool-eau qui correspondent aux volumes effectivement ob-

---

*) Les abréviations utilisées ici pour désigner des grandeurs, tels les volumes et les masses et les contractions en découlent assez directement. Ainsi par exemple $V_{aj\,mél}$ et $Vf_{mél}$ caractérisent les volumes ajoutés et apparents des mélanges et pour distinguer les différentes dilutions on parlera des mélanges 1, 2 et 3.

servés après cette addition, et que nous appellerons <u>volume apparent d'eau</u> ($V_{ap\ W}$)* ou <u>volume final apparent du mélange</u> ($Vf_{mél}$)*.

N.B.: L'effet de la contraction produira une différence entre les volumes ajoutés et apparents, de même que pour l'eau entre ($V_{aj\ W}$)* et ($V_{ap\ W.}$)*, pour les mél-anges éthanol-eau, où nous observerons une différence souvent bien notable entre le volume ajouté ($V_{aj\ mél}$)* et le volume final apparent d'un mélange donné ($Vf_{mél}$)*.

**La contraction totale:** On définit comme contraction totale, la contraction qui a lieu lors de l'addition d'une quantité d'eau à une quantité d'alcool pur, donnant un mélange alcool-eau d'une concentration donné (p. ex. 85 %vol).

**La contraction partielle:** On définit comme contraction partielle, les étapes de la contraction ayant lieu lors de la dilution avec de l'eau ou d'un mélange alcool-eau, d'un mélange alcool-eau d'une concentration donné. On distinguera alors les contractions partielles ayant lieu lors des différentes étapes de dilutions, et on parlera respectivement des contraction partielles $C_{p1}$, $C_{p2}$ et de la contraction partielle restante $C_{pr}$.

**La contraction normalisée:** On parlera de contractions normalisées lorsque celle-ci sont exprimés par rapport à volume final de mélange donné ($Vf_{mél}$)* de 100 l et on utilise alors pour les contractions les caractères $C_t$, $C_{p1}$, $C_{p2}$ et $C_{pr}$. Lorsque d'un autre côté les contractions se rapportent à un volume final ($Vf_{mél.}$)* différent de 100 l on les distinguera par une apostrophe ($C_t'$, $C_{p1}'$, $C_{p2}'$ et $C_{pr}'$).

**Les differentes manières d'exprimer les concentrations dans les mélanges d'alcool-eau**

En alcoométrie on utilise traditionnellement pour exprimer les teneurs en alcool des mélanges alcooliques, les % vol et les % mas, c'est donc par rapport à ces expressions, que la contraction a donc dans quelques cas été exprimée par rapport à l'une ou l'autre de ces deux expressions (17-21).

La signification de ces représentations en fonction des % vol et % mas est cependant limitée elle ne se réfère de manière générale, qu'aux rapports de volumes ou de masses.

Cherchant à faire apparaître du point de vue physico-chimique et moléculaire la signification de la contraction, notre attention s'est portée sur sa représentation, nouvelle pour cela, par rapport aux pour-cent molaires (% mol).

Expression en pour-cent volume (% vol)

Selon la définition officielle: *Le titre volumique (% vol)* exprimé en pour-cent du volume du mélange est le rapport entre le volume d'alcool pur et le volume du mélange, rapportés tous deux à la température de 20 °C.

Quoique d'une utilisation pratique peu aisée, les pour-cent volumes (% vol) sont cependant indispensables dans la pratique internationale de l'alcoométrie. Ainsi on calcule de manière très générale la teneur en éthanol dans tous les alcools et spiritueux sur la base de *litres d'alcool pur (litres à 100 %).*

Expression en pour-cent masse (% mas)

Selon la définition officielle: *Le titre massique (% mas)* exprimé en pour-cent de la masse du mélange est le rapport entre la masse d'alcool pur et la masse du mélange.

Il est à remarquer que les % vol, les % mas sont très fréquemment utilisés dans la pratique courante de l'alcoométrie, à laquelle dés le début, ils ont été associés

Expression en pour-cent molaires (% mol)

Alors que les % vol et les % mas expriment les rapports entre les volumes et les masses, les % mol, de leur côté expriment le rapport entre les masses molaires* de l'alcool à la somme des masses molaires dans le mélange alcool-eau (*À noter que l'expression de masse molaire utilisée ici est équivalente à celle de poids moléculaire qu'on emploie encore parfois dans la pratique).*

Les % mol et les fractions molaires ($X_{Alc}$) se calculent en relation avec les % mas de l'alcoométrie selon la formule non transformée suivante, où $M_{Alc}$ et $M_W$ représentent les masses molaires de l'éthanol et de l'eau:

---

*) Les abréviations utilisées ici pour désigner des grandeurs, tels les volumes et les masses et les contractions en découlent assez directement. Ainsi par exemple $V_{aj\ mél}$ et $Vf_{mél}$ caractérisent les volumes ajoutés et apparents des mélanges et pour distinguer les différentes dilutions on parlera des mélanges 1, 2 et 3.

$$X_{Alc} = \frac{\dfrac{\% \text{ mas} / 100}{M_{Alc}}}{\dfrac{\% \text{ mas} / 100}{M_{Alc}} + \dfrac{(1 - \% \text{ mas} / 100)}{M_W}} \qquad (1')$$

$$\% \text{ mol} = X_{Alc} \cdot 100 \qquad (1'')$$

L'expression de la contraction en fonction des % mol, utilisée ici de manière nouvelle, permet ainsi de mettre en évidence l'importance. physico-chimique de cet effet.

**Détermination du volume ajouté d'eau ($V_{aj\,W}$) en passant par la masse**

On a d'abord la formule pour le calcul de la masse volumique: " $\rho$ " (kg/m$^3$).

$$\rho\,(\text{masse volumique}) = \frac{m\,(\text{masse})}{V\,(\text{volume})} \qquad (1.1)$$

Détermination de la masse du volume final d'un mélange éthanol-eau

En utilisant la formule 1.1, on détermine d'abord la masse du mélange ($m_{\text{mél.}}$) pour un volume final ($Vf_{\text{mél.}}$) donné, qui a déjà subi l'effet de la contraction, et pour lequel on prendra souvent dans la pratique un volume de 100 l, qu'on multiplie ensuite par la masse volumique du mélange ($\rho_{\text{mèl.}}$).

$$m_{\text{mél.}} = Vf_{\text{mél}} \cdot \rho_{\text{mél}} \qquad (1.2)$$

Puis on a aussi de manière analogue:

$$m_{\text{mél.}} = m_{\text{Alc.}} + m_W \qquad (1.3)$$

Les masses d'eau et d'éthanol dans ce mélange

La masse d'éthanol se déduit comme suit de la masse du mélange alcool-eau en se basant sur la définition des pour-cent masse (% mas):

$$m_{Alc} = m_{\text{mel.}} \cdot \% \text{ mas} / 100 \qquad (1.4)$$

Pour l'eau de manière analogue on trouve:

$$m_W = m_{\text{mél.}} \cdot (1 - \% \text{ mas} / 100) \qquad (1.5)$$

N.B.: En passant par la masse, ce qu'on fait la plus part du temps dans la pratique de l'alcoométrie, les résultats ne sont pas influencés par l'effet de la contraction, à la grande différence de ce qui se passe avec les volumes.

Les volumes ajoutés d'eau et d'éthanol présents dans ce mélange:

Ces volumes, qui correspondent à la quantité effectivement ajoutée d'eau et d'éthanol, se trouvent avec l'aide des équations précédentes:

$$V_{aj\,W} = \frac{m_{\text{mét}} - m_{Alc}}{\rho_W} \qquad (1.6)$$

$$V_{aj\,Alc} = \frac{m_{mel.} - m_W}{\rho_{Alc}} \qquad (1.7)$$

Formules développées pour obtenir $V_{aj\,W}$ et $V_{aj\,Alc}$

S'obtiennent en introduisant dans 1. 6 et 1. 7, les égalités 1. 2, 1. 3, 1. 4 et 1. 5:

$$V_{aj\,W} = \frac{Vf_{mél.} \cdot \rho_{mél.} \cdot (1 - \%\,max\,/\,100)}{\rho_W} \qquad (1.8)$$

$$V_{aj\,Alc} = \frac{Vf_{mél.} \cdot \rho_{mél.} \cdot \%\,mas\,/\,100}{\rho_{Alc}} \qquad (1.9)$$

**Détermination du volume apparent de l'eau en passant par le volume**

Le volume d'alcool dans le mélange

D'abord selon la définition des pour-cent de volume (% vol), le volume d'alcool ($V_{Alc}$) dans le mélange alcool-eau s'obtient directement ainsi:

$$V_{Alc} = Vf_{mél.} \cdot \%\,vol\,/\,100 \qquad (1.10)$$

Le volume apparent d'eau dans le mélange s'obtient de même

$$V_{ap\,W} = Vf_{mél.} \cdot (1 - \%\,vol\,/\,100) \qquad (1.11)$$

N.B.: Comme montré dans le paragraphe suivant des calculs pratiques, alors que pour les volumes d'alcool, $V_{aj\,Alc}$ est égal à $V_{Alc}$, les volumes d'eau sont soumis à la contraction, ce qui fait que $V_{aj\,W} > V_{ap\,W}$. La différence appréciable entre $V_{aj\,W}$ et $V_{ap\,W}$ correspondant, pour un volume final de mélange $Vf_{mél}$ donné, à la contraction.
Les résultats des calculs montrent, que les contractions varient en fonction de la concentration des différents mélanges et passent par un maximum caractéristique.

Tableau 1:

| Valeurs des contractions (l/100 l) pour les mélanges éthanol-eau à 20 °C, en fonction de % mas, %vol, $X_{alc}$, $\rho_{mél}$ et en %mol (cf. fig. 1) | | | | | |
|---|---|---|---|---|---|
| %mas | %vol | $X_{alc}$ | %mol | Mass vol (kg/m$^3$) | Contraction à 20° |
| 0 | 0 | 0 | 0 | 998.20 | 0 |
| 2.0 | 2.52 | 0.0079 | 0.792 | 994.49 | 0.1558 |
| 5.0 | 6.27 | 0.0202 | 2.017 | 989.38 | 0.4306 |
| 7.0 | 8.75 | 0.0268 | 2.860 | 986.24 | 0.6357 |
| 10.0 | 12.44 | 0.0417 | 4.165 | 981.85 | 0.9658 |
| 12.0 | 14.89 | 0.0506 | 5.064 | 979.10 | 1.2062 |
| 15.0 | 18.53 | 0.0642 | 6.457 | 975.13 | 1.5655 |
| 17.0 | 20.95 | 0.0742 | 7.417 | 972.54 | 1.8164 |
| 20.0 | 24.55 | 0.0891 | 8.908 | 968.61 | 2.1785 |

Tableau 1: (suite)

| Valeurs des contractions (l/100 l) pour les mélanges éthanol-eau à 20 °C, en fonction de % mas, %vol, $X_{alc}$, $\rho_{mél}$ et en %mol (cf. fig. 1) | | | | | |
|---|---|---|---|---|---|
| %mas | %vol | $X_{alc}$ | %mol | Mass vol (kg/m$^3$ | Contraction à 20° |
| 22.0 | 26.92 | 0.0994 | 9.936 | 965.90 | 2.3961 |
| 25.0 | 30.46 | 0.1153 | 11.534 | 961.63 | 2.7123 |
| 27.0 | 32.79 | 0.1264 | 12.639 | 958.61 | 2.8947 |
| 30.0 | 36.25 | 0.1436 | 14.357 | 953.78 | 3.1350 |
| 32.0 | 38.53 | 0.1555 | 15.545 | 950.36 | 3.2710 |
| 35.0 | 41.90 | 0.1740 | 17.397 | 944.92 | 3.4306 |
| 37.0 | 44.12 | 0.1868 | 18.681 | 941.11 | 3.5168 |
| 40.0 | 47.39 | 0.2068 | 20.683 | 935.15 | 3.6002 |
| 42.0 | 49.55 | 0.2207 | 22.072 | 931.03 | 3.6471 |
| 45.0 | 52.72 | 0.2424 | 24.244 | 924.69 | 3.6697 |
| 47.0 | 54.81 | 0.2575 | 25.753 | 920.37 | 3.6776 |
| 50.0 | 57.89 | 0.2812 | 28.117 | 913.77 | 3.6609 |
| 52.0 | 59.91 | 0.2976 | 29.762 | 909.31 | 3.6356 |
| 55.0 | 62.90 | 0.3234 | 32.344 | 902.55 | 3.5880 |
| 57.0 | 64.85 | 0.3415 | 34.145 | 897.99 | 3.5332 |
| 60.0 | 67.74 | 0.3698 | 36.977 | 891.10 | 3.4483 |
| 62.0 | 69.64 | 0.3896 | 38.957 | 886.46 | 3.3862 |
| 65.0 | 72.43 | 0.4208 | 42.076 | 879.45 | 3.2663 |
| 67.0 | 74.26 | 0.4426 | 44.263 | 874.73 | 3.1781 |
| 70.0 | 76.95 | 0.4772 | 47.717 | 867.61 | 3.0252 |
| 72.0 | 78.71 | 0.5014 | 50.145 | 862.83 | 2.9128 |
| 75.0 | 81.31 | 0.5399 | 53.990 | 855.60 | 2.7386 |
| 77.0 | 83.00 | 0.5670 | 56.700 | 850.74 | 2.6023 |
| 80.0 | 85.49 | 0.6101 | 61.007 | 843.39 | 2.3882 |
| 82.0 | 87.11 | 0.6405 | 64.053 | 838.43 | 2.2290 |
| 85.0 | 89.48 | 0.6891 | 68.910 | 830.88 | 1.9657 |
| 87.0 | 91.02 | 0.7236 | 72.358 | 825.75 | 1.7741 |
| 90.0 | 93.27 | 0.7788 | 77.878 | 817.88 | 1.4635 |
| 92.0 | 94.71 | 0.8181 | 81.812 | 812.49 | 1.2216 |
| 95.0 | 96.79 | 0.8814 | 88.140 | 804.14 | 0.8180 |
| 97.0 | 98.12 | 0.9267 | 92.672 | 798.36 | 0.5194 |
| 100.0 | 100 0 | 1.0000 | 100.000 | 789.24 | 0 |

La contraction $"C_t"$ obtenue par la différence entre le volume ajouté et le volume apparent de l'eau

La contraction de volume $C_t$ , pour un volume donné $Vf_{mél.}$, se calcule en faisant la différence entre le volume ajouté $V_{aj\,W}$ et le volume apparent $V_{ap\,W}$ pour l'eau.

$$C_t = V_{aj\,W} - V_{ap\,W} \qquad (1.\,12)$$

N.B.: Alors que " C " représente la contraction, la petite lettre " $t$ " indique qu'il s'agit d'une contraction totale. On parlé ici de contraction totale par opposition à la contraction qui a lieu lors de l'addition d'eau à un alcool déjà dilué, où on aura alors des contractions partielles.

En divisant la formule 1. 12 par le volume final du mélange $Vf_{\text{mél}}$, on peut obtenir directement la contraction exprimée en fraction du volume final du mélange ($f\,C_t$), ce qui est parfois utile.

$$f\,C_t = (V_{aj\,W} - V_{ap\,W}) \,/\, Vf_{\text{mél.}} \qquad (1.\,13)$$

Formule développée de la contraction

Elle s'obtient introduisant les formules 1. 8 et 1. 11 dans 1. 12:

$$C_t = \frac{Vf_{\text{mél.}} \cdot \rho_{\text{mél.}} \cdot (1 - \%\,\text{mas}\,/\,100)}{\rho_W} - Vf_{\text{mél.}} \cdot (1 - \%\,\text{vol}\,/\,100) \qquad (1.14)$$

L'inventeur de la présente invention établi ainsi les développements permettant de calculer la contraction dans les mélanges éthanol-eau.

La représentation de la contraction en fonction des % mol à différentes températures

Pour arriver aux valeurs des contractions à d' autres températures que celle de 20 °C, la formule 1. 14 a été modifiée, pour tenir compte aussi des variations respectives des % vol à ces températures.

En utilisant la formule importante et souvent utilisée de la transformation des % vol en % mas, on obtiendra avec les $\rho_{\text{mél.}}\,\rho_{\,\text{Alc}}$ pour une température et un % mas donné, des % vol à une température [T] donnée, s'écartant ainsi des % vol, qui selon la définition officielle sont rapportées à 20 °C.

$$\%\,\text{vol}_{\,T} = \frac{\%\,\text{mas} \cdot \rho_{\text{mél}\,T}}{\rho_{\text{Alc}\,T}} \qquad (1.\,15)$$

En remplaçant ensuite la formule 1. 15 dans 1. 14 on obtiendra pour la contraction la formule générale suivante, exprimée en fonction des % vol $_T$.

$$C_{t\text{T}}' = \frac{Vf_{\text{mél}\,T} \cdot \rho_{\,\text{mél}\,T} \cdot (1 - \%\,\text{mas}\,/\,100)}{\rho_{\,WT}} - Vf_{\text{mél}\,T} \cdot (1 - \%\,\text{mas} \cdot \rho_{\,\text{mél}\,T}\,/\,100 \cdot \rho_{\,\text{Alc}\,T}) \qquad (1.\,16)$$

**N.B.:** Les formules générales 1. 15 et 1. 16 deviennent spécifiques lorsque T = 20 °C et qu'alors les % vol$_T$ sont égales aux % vol des tables alcoo-métriques. Dans 1.16 non rapportés à 100 l et de ce fait non normalisée l'apostrophe a aussi été ajouté ici à $C_t$ et $C_{t\text{T}}$.

La contraction ($C_t$) à des températures de 0 à 30 °C a donc pu être calculée à l'aide de la formule 1. 16, qui découle comme montré avant de 14. Les données pour les masses volumiques $\rho_{\text{mél.}}$, $\rho_W$ et $\rho_{\text{Alc}}$ pour ces différentes températures en fonction des % mas sont celles des Tables alcoométriques internationales de l' OIML (22,23). Les valeurs des contractions à ces différentes températures, couvrant le domaine entier des concentrations des mélanges éthanol-eau exprimées en fonction des % mol sont réunies dans le tableau 2 et représentées dans figure 2.

Tableau 2:

| Valeurs des contractions (l/100 l) pour les mélanges éthanol-eau en fonction de % mas et $X_{\text{alc}}$ à 5, 10, 15 et 20 °C (cf. fig. 2) | | | | | |
|---|---|---|---|---|---|
| % mas | $X_{\text{alc}}$ | contraction à 5°C | contraction à 10 °C | Contraction à 15 °C | contraction à 20 °C |
| 0 | 0 | 0 | 0 | 0 | 0 |

Tableau 2: (suite)

| % mas | $X_{alc}$ | contraction à 5°C | contraction à 10 °C | Contraction à 15 °C | contraction à 20 °C |
|---|---|---|---|---|---|
| Valeurs des contractions (l/100 l) pour les mélanges éthanol-eau en fonction de % mas et $X_{alc}$ à 5, 10, 15 et 20 °C (cf. fig. 2) | | | | | |
| 5 | 0.0202 | 0.3585 | 0.3824 | 0.4063 | 0.4285 |
| 10 | 0.0417 | 0.8853 | 0.9139 | 0.9408 | 0.9663 |
| 15 | 0.0646 | 1.5353 | 1.5460 | 1.5572 | 1.5685 |
| 20 | 0.0891 | 2.2295 | 2.2062 | 2.1888 | 2.1739 |
| 25 | 0.1153 | 2.8713 | 2.8099 | 2.7589 | 2.7129 |
| 30 | 0.1436 | 3.3862 | 3.2922 | 3.2119 | 3.1394 |
| 35 | 0.1740 | 3.7388 | 3.6241 | 3.5236 | 3.4344 |
| 40 | 0.2068 | 3.9328 | 3.8111 | 3.7027 | 3.6051 |
| 45 | 0.2424 | 3.9981 | 3.8777 | 3.7699 | 3.6726 |
| 50 | 0.2812 | 3.9670 | 3.8544 | 3.7528 | 3.6601 |
| 55 | 0.3234 | 3.8643 | 3.7620 | 3.6700 | 3.5843 |
| 60 | 0.3698 | 3.7037 | 3.6116 | 3.5294 | 3.4519 |
| 65 | 0.4208 | 3.4897 | 3.4081 | 3.3347 | 3.2657 |
| 70 | 0.4772 | 3.2217 | 3.1519 | 3.0875 | 3.0261 |
| 75 | 0.5399 | 2.9024 | 2.8423 | 2.7871 | 2.7346 |
| 80 | 0.6101 | 2.5250 | 2.4759 | 2.4302 | 2.3870 |
| 85 | 0.6891 | 2.0767 | 2.0400 | 2.9977 | 2.9702 |
| 90 | 0.7788 | 1.5341 | 1.5086 | 1.4834 | 1.4595 |
| 95 | 0.8814 | 0.8558 | 0.8488 | 0.8345 | 0.8214 |
| 100 | 1.0000 | 0 | 0 | 0 | 0 |

Dans le graphique (cf. fig. 2) on peut ainsi voir des courbes de contraction de 0 à 30 °C représentées en fonction des % mol, qui se ressemblent, mais sont cependant bien distinctes les unes des autres. On peut aussi voir que la contraction augmente lorsque diminue la température.

Il faut cependant remarquer, que cette augmentation de la contraction en fonction de la température est maximum dans le domaine des % mol pour lesquelles la contraction passe également par un maximum, alors qu'elle est beaucoup plus faible voire imperceptible dans les domaines extérieures de la courbe.

En observant la contraction pour les mélanges éthanol-eau, vers le maximum de la contraction, on peut noter que l'augmentation est très nette, puisqu'elle passe pour un $X_{Alc}$ de 0,242, d'une contraction pour 100 l de 3,672 l à 20 °C à une contraction de 3,998 l à 5 °C, ce qui représente une augmentation de 0,326 l ou 8,9 % par rapport à la contraction totale.

La contraction partielle lors de la dilution de mélanges alcool- eau ou d'eaux-de-vie par addition d'eau

Pour examiner en détail comment se calculent et se représentent les contractions partielles et totales, on va s'intéresser ici à la dilution de solutions alcooliques. La dilution de mélanges alcool-eau ou eaux-de-vie avec de l'eau, étant un mode de dilution très couramment utilisé dans la pratique.

Si l'on fixe un volume déterminé pour le mélange initial ($Vf_{mél.\ 1}$) on parlera ici de *la méthode 1a*; si par contre on fixe un volume déterminé pour le mélange final ($Vf_{mél.\ 2}$) on parlera de *la méthode 1b.*

**Méthode 1a : Dilution par addition d'eau en partant d'un volume fixe déterminé de mélange ($Vf_{mél. 1}$)**

***Calcul du volume d'eau à ajouter à un mélange alcool-eau* fixe *déterminé pour obtenir un mélange final d'une teneur en alcool recherchée***

Dans la pratique de l'alcoométrie on est souvent appelé à calculer le nombre de litres d'eau à rajouter ($V_{aj\,W}$) à un volume fixe déterminé d'un alcool ou une eau-de-vie ($Vf_{mél. 1}$) d'une teneur en alcool en $x_1$ et de masse volumique $\rho_1$ pour obtenir un alcool ou une eau-de-vie d'une teneur en alcool finale $x_2$ ( où $x_1$ et $x_2$ sont exprimés en % mas).
On utilise pour cela une formule pratique suivante, dans laquelle on fixe pour $Vf_{mél. 1}$ un volume de 100 l (26):

$$V_{aj\,W} = \frac{100\,(l) \cdot \rho_{mél\,1} \cdot (x_1/x_2 - 1)}{\rho_W} \tag{2.1}$$

Dans la pratique la détermination de ce volume d'eau à ajouter (Vaj W ) peut aussi se faire avec l'aide de tables établies spécialement pour cela et qui ont été calculés avec la formule 2.1 (26).
Comme expliqué dans les paragraphes suivants ces valeurs recalculés ici ont été complétées de manière nouvelle en tenant compte des valeurs spécifiques de la contraction partielle ( cf. table 3).
En procédant selon ces calculs, on peut déterminer la teneur en alcool exacte du mélange que l'on veut obtenir et le volume ajouté (Vaj mél. 2), mais sans pour autant connaître avec précision le volume final (Vf mél. 2), car on tient ainsi compte, comme cela sera montré dans la figure 3, que de manière incomplète de la contraction. Pour cela on va développer ici les raisonne-ments sur lesquels se basent ces calculs.
Ainsi en passant par la masse, on peut appliquer les formules suivantes qui dérivent des formules plus générales 4.1 et 4.2 :

$$m_W = m_{mél\,2} - m_{mél.\,1} \tag{2.2}$$

Et d'où la partie gauche de l'équation devenant nulle:

$$[m_W \cdot 0 = m_{mél.\,2} \cdot x_2 - m_{mél.\,1} \cdot x_1]$$

$$m_{mél.\,1} \cdot x_1 = m_{mél\,2} \cdot x_2$$

et puis :

$$m_{mél\,2} = m_{mél.1} \cdot x_1 / x_2 \tag{2.3}$$

En utilisant la formule de la masse volumique
[ p = masse / volume ] et en remplaçant la formule 2.3 dans 2.2 on obtient :

$$V_{aj\,W} \;\; \rho_W = Vf_{mél.1} \cdot \rho_{mél\,1} \cdot x_1 / x_2 - Vf_{mél1} \cdot \rho_{mél\,1} \tag{2.4}$$

$$\underbrace{\quad}_{\text{masse de H2O}} \quad \underbrace{\quad}_{\text{masse du mélange final}} \quad \underbrace{\quad}_{\text{masse du mélange initial}}$$

De là l'on tire :

$$V_{aj\,W} = \frac{Vf_{mél.\,1} \cdot \rho_{mél\,1} \cdot x_1 / x_2 - Vf_{mél\,1} \cdot \rho_{mél\,1}}{\rho_W} \tag{2.5}$$

La formule 2. 5 correspond donc à la formule 2.1 en remplaçant le volume du mélange initial ($Vf_{mel1}$) par les 100 l utilisés habituellement dans la pratique.

N.B.: Il est important de tenir compte, comme montré avant (cf. fig. 2 et table 2), que la contraction est variable en fonction de la température et que si la température du mélange initial s'écarte sensiblement de la <u>température de référence de 20° C,</u> les corrections des grandeurs: $Vf_{mél.1}$, $\rho_{mél 1.}$ $x_1$, sont indispensables en vue de résultats corrects.

### *Calcul des contractions partielles lors de dilutions selon la méthode 1a*

En ajoutant le volume d'eau à compléter ($V_{aj\,W}$), au volume du mélange de départ ($Vf_{mél\,1}$), on obtient le volume ajouté du mélange 2 ($V_{aj\,mél.2}$), qui ne tient pas compte de la contraction ayant lieu à ce stade (cf. table 3, fig. 3) :

$$V_{aj\,mél\,2} = Vf_{mél\,2} + V_{aj\,W} \qquad\qquad (2.6)$$

D'un autre côté nous obtenons comme suit, de manière analogue à 2.3 le volume final apparent du mélange :

$$Vf_{mél\,2} = Vf_{mél\,1} \cdot y_1 / y_2 \qquad\qquad (2.7)$$

Où $y_1$ et $y_2$ expriment respectivement en % vol les teneurs en alcool du mélange $Vf_{mél.1}$ et $Vf_{mél.2}$. De la on peut déduire la contraction partielle restante non normalisée ($C_{pr}'$) ayant lieu lors de cette dilution :

$$C_{pr}' = V_{aj\,mél.2} - Vf_{mél\,2} \qquad\qquad (2.8)$$

N. B.: Il est à remarquer qu'il ne s'agit ici que d'une contraction partielle restante ($C_{pr}'$), car le mélange initial a une teneur en alcool ($y_1$), qui a déjà été soumis à une contraction partielle primaire ($C_{p1}'$). D'autre part si ces contractions partielles sont marquées d'une apostrophe cela veut dire que le volume final $Vf_{mél\,2}$ n'est pas encore, normalisée à 100 l.

<u>Table 3</u> : Table de dilution selon la méthode 1a, donnant en fonction d'un volume déterminé fixe de 100 l de mélanges alcool-eau de 85 à 40 % vol ($Vf_{mél.\,1}$), les volumes d'eau ajoutés ($V_{aj\,W}$), les volumes d'eau apparents ($V_{ap\,W}$) et les contractions partielles restantes ($C_{pr}'$) exprimées par rapport au volume final apparent ($Vf_{mél.\,2}$).

## Table 3

| Teneur de 100 l Vf mél.1 | | m. vol. (kg/m³) | 60 % vol (Vf mél. 2) | | | 50 % vol (Vf mél. 2) | | | 40 % vol (Vf mél. 2) | | |
|---|---|---|---|---|---|---|---|---|---|---|---|
| % vol | % mas | | Vaj w | Vap w | Cpr' | Vaj w | Vap w | Cpr' | Vaj w | Vap w | Cpr' |
| 85.0 | 79.40 | 844.85 | 44.374 | 41.667 | 2.707 | 73.746 | 70.000 | 3.746 | 117.171 | 112.500 | 4.671 |
| 82.0 | 75.82 | 853.62 | 38.957 | 36.667 | 2.291 | 67.296 | 64.000 | 3.296 | 109.193 | 105.000 | 4.193 |
| 80.0 | 73.48 | 859.27 | 35.348 | 33.333 | 2.015 | 62.994 | 60.000 | 2.994 | 103.867 | 100.000 | 3.867 |
| 77.0 | 70.06 | 867.48 | 29.980 | 28.333 | 1.647 | 56.591 | 54.000 | 2.591 | 95.934 | 92.500 | 3.434 |
| 75.0 | 67.82 | 872.79 | 26.404 | 25.000 | 1.404 | 52.322 | 50.000 | 2.322 | 90.640 | 87.500 | 3.140 |
| 72.0 | 64.53 | 880.54 | 21.067 | 20.000 | 1.067 | 45.946 | 44.000 | 1.946 | 82.729 | 80.000 | 2.729 |
| 70.0 | 62.39 | 885.56 | 17.542 | 16.667 | 0.876 | 41.734 | 40.000 | 1.734 | 77.500 | 75.000 | 2.500 |
| 67.0 | 59.22 | 892.89 | 12.244 | 11.667 | 0.577 | 35.396 | 34.000 | 1.396 | 69.626 | 67.500 | 2.126 |
| 65.0 | 57.15 | 897.65 | 8.735 | 8.333 | 0.402 | 31.198 | 30.000 | 1.198 | 64.407 | 62.500 | 1.907 |
| 62.0 | 54.09 | 904.60 | 3.479 | 3.333 | 0.146 | 24.904 | 24.000 | 0.904 | 56.578 | 55.000 | 1.578 |
| 60.0 | 52.09 | 909.11 | 0.000 | 0.000 | 0.000 | 20.735 | 20.000 | 0.735 | 51.390 | 50.000 | 1.390 |
| 57.0 | 49.13 | 915.70 | | | | 14.486 | 14.000 | 0.486 | 43.609 | 42.500 | 1.109 |
| 55.0 | 47.18 | 919.96 | | | | 10.317 | 10.000 | 0.317 | 38.415 | 37.500 | 0.915 |
| 52.0 | 44.31 | 926.16 | | | | 4.111 | 4.000 | 0.111 | 30.677 | 30.000 | 0.677 |
| 50.0 | 42.43 | 930.14 | | | | 0.000 | 0.000 | 0.000 | 25.548 | 25.000 | 0.548 |
| 47.0 | 39.64 | 935.88 | | | | | | | 17.850 | 17.500 | 0.350 |
| 45.0 | 37.80 | 939.54 | | | | | | | 12.719 | 12.500 | 0.219 |
| 42.0 | 35.09 | 944.76 | | | | | | | 5.088 | 5.000 | 0.088 |
| 40.0 | 33.30 | 948.05 | | | | | | | 0.000 | 0.000 | 0.000 |

La formule 2.8 peut être développée en y remplaçant les équations 2.6 et 2.7.

$$C_{pr}' = Vf_{mél.1} + V_{aj\,W} - Vf_{mél.1} \cdot y_1 / y_2 \qquad (2.9)$$

De là on obtient :

$$C_{pr}' = V_{aj\,W} - Vf_{mél.1} \cdot (y_1 / y_2 - 1) \qquad (2.10)$$

Puis on obtient la formule plus générale en remplaçant $V_{aj\,W}$ par la formule 2.5.

$$C_{pr}' = \frac{Vf_{mél.1} - \rho_{mél\,1} \cdot (x_1 / x_2 - 1)}{\rho_W} - Vf_{mél.1}\,(y_1 / y_2 - 1) \qquad (2.11)$$

On pourra calculer la contraction totale ($C_t'$) du mélange comme suit :

$$C_t' = C_{p1}' + C_{pr}' \qquad (2.12)$$

La contraction partielle ($C_{p1}'$) se calculant en faisant appel à la formule générale donnée pour cela auparavant (1.14 ou 1.16).

***Exemple de référence pratique mettant en évidence l'effet de la contraction lors d'une dilution selon la méthode 1a :***

En partant d'un mélange alcool-eau à un volume et une teneur en alcool donnée, on veut obtenir n litres d'un alcool à 40 % vol, à la température de référence de 20 °C.

Comme montré aussi dans l'exemple suivant, comme les différents paramètres varient en fonction de la température, il est important de faire les corrections nécessaires pour les ramener avant à 20 °C.

En partant ainsi d'un mélange alcool-eau, pour lequel à la température de 6 °C un volume de 1973,2 l et 81 % vol ont été mesurés, on devra d'abord, selon les méthodes usuelles en alcoométrie, calculer le % vol et le volume final apparent ($Vf_{mél.1}$) à la température de référence de 20 °C (23-28). Ensuite seulement on pourra déterminer les volumes ajoutés ($V_{aj\,W}$) et ($V_{aj\,mél.2}$), puis le volume final apparent ($Vf_{mél.2}$) et enfin la contraction totale et partielle pour le mélange.

Ainsi ce volume de 1973,2 l à 81 % vol mesuré à 6 °C, donnera après correction avec les tables officielles (26,29,30) 2000 l à 85 % vol à la température de référence de 20 °C.

En appliquant ensuite la formule 2.1 / 2.5 avec $Vf_{mél.1}$ = 2000 l, à 85,0 % vol ($x_1$ : 73,48 % mas, $\rho_{mél.1}$ = 844,85 kg /m$^3$) qu'on veut diluer à 40 % vol ($x_2$ : 33,3 % mas, $\rho_{mél.2}$ = 948,05 kg /m$^3$), en ajoutant de l'eau, $\rho_W$ = 998,20 (kg /m$^3$), on obtient le volume ajouté d'eau suivant:

$$V_{aj\,W} = 2343,41 \text{ l}$$

De la on tire selon 2.6 le volume ajouté final :

$$V_{aj\,mél\,2} = 2000 \text{ l} + 2343,41 \text{ l} = 4343,41 \text{ l}$$

D'autre part on peut ainsi calculer le volume final apparent du mélange 2 :

$$Vf_{mél.2} = 2000 \text{ l} \cdot \frac{85 \text{ % vol /100}}{40 \text{ % vol / 100}} = 4250 \text{ l}$$

De là l'on obtient selon 2.8 la contraction partielle suivante pour ces 4250 l:

$$C_{pr}' = 4343,41 \text{ l} - 4250 \text{ l} = 93,41 \text{ l}$$

Normalisé à un volume final apparent ($Vf_{mél.2}$) de 100 l cela donne :

$$C_{pr} = 2{,}198 \text{ l}$$

Cependant comme au départ on avait déjà un $Vf_{mél.1}$ de 2000 l à 85 % vol et à 20 °C, qui avait déjà été soumis à une contraction totale, en introduisant les paramètres de cet exemple, comme indiqués auparavant, dans la formule 1.14 on obtient pour ce volume de 2000 l la contraction partielle suivante :

- Il est utile de remarquer à ce sujet, que lorsqu'on applique la formule générale 1.14 on calcule une contraction totale pour le mélange 1 donné, ce qui correspond à une contraction totale ($C_t'$). Mais qu' une fois ramenée au volume du mélange 2, ici 4250 l, cette même contraction devient une contraction partielle ($C_{p1}'$).
- Aussi dans des cas d'écarts de température il faut veiller à ce que l'eau de réduction soit à la même température que celle du mélange à diluer.

Ce volume de 48,706 l est donc la contraction à prendre en considération pour 4250 l et qui ramené à 100 l ($Vf_{mél.1}$) équivaut à :

$$C_{p1} = 1{,}146 \text{ l.}$$

Dans ce cas nous obtenons donc selon la formule 2. 12 la valeur de la contraction totale ($C_t$) :

$$C_t' = 93{,}41 \text{ l} + 48{,}706 \text{ l} = 142{,}21 \text{ l}$$

Ce qui normalisé à un $Vf_{mél.2}$ de 100 l équivaut à :

$$C_t = 1{,}146 \text{ l} + 2{,}198 \text{ l} = 3{,}344 \text{ l}$$

Dans l'exemple choisi ici il est aussi à noter que le volume fixe déterminé de 2000 l est un volume final apparent ($Vf_{mél.1}$) et non un volume ajouté ($V_{aj\ mél.1}$), puisque la contraction y est déjà prise en compte dans la méthode de calcul.
On peut d'un autre côté observer que les contractions ainsi déterminée correspond bien aux valeurs indiquées pour 40 % vol dans la figure 5 donnant les contractions pour des mélanges éthanol-eau de 100 l en fonction des teneurs en alcool exprimés en % vol.
Dans la fig. 3 et le tab. 3 est examiné le cas ou de l'eau est ajoutée pour diluer un volume ($Vf_{mél\ 1}$) fixe déterminé de 100 l à une teneur de 85 %vol (75 et 65 % vol), pour obtenir selon la méthode 1a (21, 27) le volume ajouté $V_{aj\ mél\ 2}$ d'une teneur en alcool recherchée. La nouvelle mise en évidence du volume $Vf_{mél.2}$ également représenté dans la figure 3 et tableau 3, rend bien visible la différence qui existe entre ces deux valeurs et qui est égale à la contraction pour le mélange 2 ($C_{pr}'$). Comme les volumes $Vf_{mél.2}$ obtenus dans ce cas sont différents de 100 l, ils doivent donc être ramenés à ce volume avant de pouvoir être comparé aux contractions données dans fig. 5. Il est à remarquer qu'une bonne coïncidence entre valeurs calculées et graphiques est ainsi observée.

**Indications d'associations moléculaires dans les mélanges éthanol-eau**

Les mélanges alcool-eau ayant été étudiés par les techniques tels les ultrasons, la RMN, l'anisotropie de la fluorescence, la détermination de la viscosité etc. (1-8) la possibilité de formation d'associations-moléculaires, avec pour les mélanges éthanol-eau un maximum situé dans la région de 20 à 25 % mol, a été à plusieurs reprises discutée.
Ainsi les résultats des mesures ultrasoniques de mélanges éthanol-$H_2O$ sont interprétés par Bruun et al. (1) comme indication de la formation de " ice-like water structures " autour du groupe non-polaire de l'alcool. La formation de l'équilibre :

$$A\,(H_2O)_n \qquad A + nH_2O$$

avec n = 3 - 4 pour $X_{Alc}$ = 0,2 - 0,3 est aussi envisagée dans ce contexte.
Avec leurs résultats de l'étude par "picosecond fluorescence anisotropy" de mélanges éthanol-eau, G. S. Beddard et al. (5, 6) supportent les models de "hydrophobic hydration", proposé précédemment sur la base de données thermodynamiques et spéctroscopiques. Dans ce contexte il est proposé par ces auteurs, qu'avec une viscosité croissante jusqu' à $X_{Alc}$ de 0,2, les molécules d'éthanol sont considérées comme étant logées dans les interstices de la structure à trois dimensions de l'eau. La partie hydrophobe de l'éthanol étant entourée de cages d'eau alors que le groupe hydroxyle remplace un des $H_2O$. Pour des $X_{Alc}$ supérieurs à 0,2, les molécules d'alcool ne trouvent plus place dans la structure de l'eau, qui est ensuite désagrégée, en ouvrant les liaisons hydrogènes entre les $H_2O$, ce qui aurait pour effet une diminution de la viscosité du mélange.

Les résultats observés dans ce travail en ce qui concerne la contraction dans les mélanges éthanol-eau, avec un maximum vers 0,20 - 0,25 % mol viennent donc appuyer, par des constations venant d'une toute autre direction et par des mesures d'un autre type, les résultats ou hypothèses des travaux précédents.

Pour donner plus de précision à nos observations sur les rapports moléculaire d'éthanol et d'eau en présence, nous avons encore étudié, comme discuté dans le paragraphe suivant, les courbes des contractions pour les mélanges éthanol-eau en fonction des % mol à l'aide de la " méthode des variations continues ".

Étude de l' application de la méthode des variations continues

Lorsque la contraction est représentée en fonction des $X_{Alc}$ ou des % mol, les maxima très caractéristiques observés permettent d'envisager la possibilité de l' application de la " méthode des variations continues".

Dans ce contexte il est nécessaire de préciser: La" *méthode des variations continues* ", dont l'application est passée en revue par *Hill* et *Mac Carty* (11) et dont le principe avait déjà été proposé longtemps avant par *Ostrmisslensky* en 1911 (12) et *Denison* en 1912 (9) est bien connue aussi sous le nom de " *méthode de Job* " (10), auteur ayant publié en 1928 une application détaillée de cette méthode aux composés de coordination.

Si en règle générale la méthode des variations continues et d'autres méthodes analogues sont appliquées aux complexes forts présents en petites concentrations, comme par exemples lors de l'étude de complexes du bore par *Liebich* et *Marcantonatos* (14), par contre leur application, à des mélanges très concentrés, comme ceux d'alcool-eau, comme déjà envisagé par *Denison* (9), est à notre connaissance très peu connue et utilisé.

En examinant attentivement les mélanges éthanol-eau, variant de 0% à 100% mol dans le cadre des conditions requises par la méthode de *Job,* aucune raison pouvant s'opposer à l'utilisation de cette méthode n'a pu être constatée.

Par analogie à la méthode de *Job,* on établit donc une équation pour l'équilibre des molécules d'alcool et d'eau, en utilisant respectivement pour ces deux composés les lettres A et W et les coefficients i et j:

$$A_i + W_j \; A_i \, W_j \qquad\qquad (2.\,1')$$

D'autre part la méthode des variations continues devrait également permettre, par l'intermédiaire de la courbe de la contraction, de déterminer le rapport i / j selon la formule suivante:

$$i\,/\,j = X_{Alc\,max}\,/\,X_{W\,max} = X_{Alc\,max}\,/\,(1 - X_{Alc\,max}) \qquad\qquad (2.\,2')$$

D'où l'on tire:

$$X_{Alc\,max} = i\,/\,i + j \qquad\qquad (2.\,3')$$

Pour les mélanges éthanol-eau pour lesquels les maximum de la contraction observés se situent entre $X_A$ = 0,25 et 0,20 on peut faire les hypothèses suivantes:

a) Pour $X_{Alc}$ = 0,25 on aurait donc : i / i + j = 1 / 4 et par suite i / j = 1 / 3 ce qui correspondrait à l' association: $A_i \, W_j = A_1 \, W_3$

b) Pour $X_{Alc}$ = 0,20 cela aurait d'un autre côté aurait donné: i / j = 1 / 4 et $A_i W_j = A_1 \, W_4$

Cependant avec l'application de la " technique de l'extrapolation " aussi parfois utilisée dans le cadre de la méthode des variations continues (cf. fig. 2) (9-11), et que nous avons aussi utilisée ici pour tenter d'améliorer l'interprétation des courbes des contractions, nous avons pu mettre en évidence que le maximum de la contraction se situait entre un $X_{Alc}$ de 0,25 et 0,20. Lors de cette extrapolation, utilisée pour déterminer le rapport molaire recherché, on prend la valeur de $X_{Alc}$ correspondant à l'intersection des deux tangentes appliquées aux deux extrémités de la courbe de la contraction. Si ici des valeurs de $X_{Alc}$ entre 0,25 et 0,20 sont obtenus, c'est aussi en raison d'une inflexion située du côté des rapports molaires faibles en éthanol, dont il a été tenu compte. Il est à remarquer qu'une telle inflexion, comme montré dans certains travaux (13, 14), peut aussi être un indice de polynucléarité.

Dans ces conditions une association moléculaire plus compliquée, avec $X_{Alc}$ situé entre 0,25 et 0,20 , comme par exemple 0,222 correspondant à une associa-tion de type $A_2W_7$ peut donc également être envisagée. Des rapports éthanol-eau d'une certaine complexité sont ainsi par exemple montrés par la diffraction de neutrons du composé moléculaire" cyclodextrine-ethanol octahydrate" (15).

Table 4 : Table de dilution selon la méthode 1b pour obtenir 100 l de mélange alcool-eau final apparent ($Vf_{mél\ 2}$) entre 85 et 10 % vol, donnant les volumes d'eau ajoutés ($V_{aj\ W}$) puis les volumes des mélanges alcool-eau ajoutés ($V_{aj\ mél.1}$) à 85, 75 et 65 % vol, ainsi que les contractions partielles restantes ($C_{pr}$) exprimées directement par rapport à 100 l ($Vf_{mél.2}$).

Table 4

| Teneurs de *Vf mél. 2* (100 l) | | | *Vaj w* | *Vap w* | *Vfmél 1* | *Cpr* | *Vaj w* | *Vap w* | *Vfmél 1* | *Cpr* | *Vaj w* | *Vap w* | *Vfmél 1* | *Cpr* |
|---|---|---|---|---|---|---|---|---|---|---|---|---|---|---|
| % vol | % mas | m. vol. (kg/m³) | 85 % vol / *Vf mél.1* | | | | 75 % vol / *Vf mél.1* | | | | 65 % vol / *Vf mél.1* | | | |
| 85.0 | 79.40 | 844.85 | 0.000 | 0.000 | 100.000 | 0.000 | | | | | | | | |
| 82.0 | 75.82 | 853.62 | 3.856 | 3.529 | 96.471 | 0.326 | | | | | | | | |
| 80.0 | 73.48 | 859.27 | 6.418 | 5.882 | 94.118 | 0.536 | | | | | | | | |
| 77.0 | 70.06 | 867.48 | 10.223 | 9.412 | 90.588 | 0.811 | | | | | | | | |
| 75.0 | 67.82 | 872.79 | 12.752 | 11.765 | 88.235 | 0.987 | 0.000 | 0.000 | 100.000 | 0.000 | | | | |
| 72.0 | 64.53 | 880.54 | 16.520 | 15.294 | 84.706 | 1.226 | 4.279 | 4.000 | 96.000 | 0.279 | | | | |
| 70.0 | 62.39 | 885.56 | 19.006 | 17.647 | 82.353 | 1.359 | 7.103 | 6.667 | 93.333 | 0.436 | | | | |
| 67.0 | 59.22 | 892.89 | 22.734 | 21.176 | 78.824 | 1.558 | 11.343 | 10.667 | 89.333 | 0.676 | | | | |
| 65.0 | 57.15 | 897.65 | 25.200 | 23.529 | 76.471 | 1.670 | 14.148 | 13.333 | 86.667 | 0.815 | 0.000 | 0.000 | 100.000 | 0.000 |
| 62.0 | 54.09 | 904.60 | 28.888 | 27.059 | 72.941 | 1.829 | 18.346 | 17.333 | 82.667 | 1.013 | 4.852 | 4.615 | 95.385 | 0.237 |
| 60.0 | 52.09 | 909.11 | 31.326 | 29.412 | 70.588 | 1.914 | 21.124 | 20.000 | 80.000 | 1.124 | 8.064 | 7.692 | 92.308 | 0.371 |
| 57.0 | 49.13 | 915.70 | 34.973 | 32.941 | 67.059 | 2.031 | 25.281 | 24.000 | 76.000 | 1.281 | 12.873 | 12.308 | 87.692 | 0.566 |
| 55.0 | 47.18 | 919.96 | 37.399 | 35.294 | 64.706 | 2.105 | 28.048 | 26.667 | 73.333 | 1.381 | 16.078 | 15.385 | 84.615 | 0.693 |
| 52.0 | 44.31 | 926.16 | 41.004 | 38.824 | 61.176 | 2.181 | 32.163 | 30.667 | 69.333 | 1.497 | 20.846 | 20.000 | 80.000 | 0.846 |
| 50.0 | 42.43 | 930.14 | 43.387 | 41.176 | 58.824 | 2.211 | 34.885 | 33.333 | 66.667 | 1.551 | 24.001 | 23.077 | 76.923 | 0.924 |
| 47.0 | 39.64 | 935.88 | 46.949 | 44.706 | 55.294 | 2.243 | 38.957 | 37.333 | 62.667 | 1.624 | 28.726 | 27.692 | 72.308 | 1.034 |
| 45.0 | 37.80 | 939.54 | 49.314 | 47.059 | 52.941 | 2.255 | 41.663 | 40.000 | 60.000 | 1.663 | 31.869 | 30.769 | 69.231 | 1.099 |
| 42.0 | 35.09 | 944.76 | 52.818 | 50.588 | 49.412 | 2.230 | 45.676 | 44.000 | 56.000 | 1.676 | 36.534 | 35.385 | 64.615 | 1.149 |
| 40.0 | 33.30 | 948.05 | 55.143 | 52.941 | 47.059 | 2.202 | 48.342 | 46.667 | 53.333 | 1.676 | 39.636 | 38.462 | 61.538 | 1.174 |
| 30.0 | 24.61 | 962.21 | 66.517 | 64.706 | 35.294 | 1.811 | 61.416 | 60.000 | 40.000 | 1.416 | 54.885 | 53.846 | 46.154 | 1.039 |
| 20.0 | 16.21 | 973.56 | 77.620 | 76.471 | 23.529 | 1.149 | 74.220 | 73.333 | 26.667 | 0.887 | 69.868 | 69.231 | 30.769 | 0.637 |
| 10.0 | 8.01 | 984.71 | 88.697 | 88.235 | 11.765 | 0.461 | 86.998 | 86.667 | 13.333 | 0.331 | 84.822 | 84.615 | 15.385 | 0.207 |

EP 1 112 992 B1

**Méthode 1b : Dilution par addition d'eau pour obtenir un volume fixe déterminé de mélange final ($Vf_{mél.\ 2}$)**

**Calcul du volume d'eau et du volume d'un mélange alcool-eau donné à ajouter l'un à l'autre pour obtenir un mélange final d'un volume déterminé et d'une teneur en alcool recherchéée.**

Nous proposons ici une méthode à notre connaissance peu utilisée dans la pratique de l'alcoométrie (21), mais qui paraît cependant d'une grande utilité. Il s'agit ainsi de déterminer le volume d'eau à ajouter ($V_{aj\ W}$) au volume d'un mélange alcool-eau ou une eau-de-vie d'une teneur en alcool $x_1$ et de masse volumique $\rho_1$ pour obtenir un volume déterminé, par exemple 100 l, d'un mélange d'alcool ou d'eau-de-vie d'une teneur en alcool finale $x_2$ (avec $x_1$ et $x_2$ exprimés en % mas).

De manière un peu analogue au développement fait pour la méthode 1a, on pose l'égalité suivante :

$$V_{aj\ W} \cdot \rho_W = Vf_{mél.2} \cdot \rho_{mél\ 2} \quad - \quad Vf_{mél2} \cdot \rho_{mél.2} \cdot x_2 / x_1 \qquad (3.1)$$

masse de $H_2O$ — masse du mélange final — masse du mélange initial

D'où l'on tire :

$$V_{aj\ W} = \frac{Vf_{mél\ 2} \cdot \rho_{mél\ 2} - Vf_{mél.2} \cdot \rho_{mél\ 2} \cdot x_2/x_1}{\rho_W} \qquad (3.2)$$

De 3.2, on parvient à 3.3 en remplaçant le volume de mélange final ($Vf_{mél.2}$) par un volume déterminé fixé à 100 l, et on obtient ainsi la formule simplifiée:

$$V_{aj\ W} = \frac{100\ (l) \cdot \rho_{mél\ 2} \cdot (1 - x_2/x_1)}{\rho_W} \qquad (3.3)$$

La détermination de ce volume d'eau à ajouter ($V_{aj\ W}$) peut se faire aussi avec l'aide de tables qui seront établies spécialement, et qui ont été calculée ici de manière toute nouvelle avec la formule 3.3 à titre d'extrait ( cf. la table 4). On peut aussi se représenter la manière dont fonctionne la méthode 1b avec la figure 4.

**Calcul des contractions partielles lors de la dilution selon la méthode 1b**

D'abord nous obtenons comme suit le volume du mélange initial :

$$Vf_{mél\ 1} = Vf_{mél.2} \cdot y_2 / y_1 \qquad (3.4)$$

En enlevant le volume du mélange initial ($Vf_{mél\ 1}$) au volume du mélange final ($Vf_{mél\ 2}$), on obtient le volume apparent de l'eau ($V_{ap\ W}$) qui ne tient pas compte de la contraction :

$$V_{ap\ W} = Vf_{mél.2} - Vf_{mél.1} \qquad (3.5)$$

Ce qui donne ensuite :

$$V_{ap\ W} = Vf_{mél\ 2} - Vf_{mél.2} \cdot y_2 / y_1 \qquad (3.6)$$

Ainsi avec l'aide des équations 3.3 et 3.6 nous obtenons la contraction partielle restante ($C_{pr}$)

$$C_{pr} = V_{aj\ W} - V_{ap\ W} \qquad (3.7)$$

Ce qui donne la formule développée suivante :

$$C_{pr} = \frac{Vf_{mél.2} \cdot \rho_{mél\,2} \cdot (1 - x_2/x_1)}{\rho W} - Vf_{mél\,2}\,(1 - y_2/y_1) \qquad\qquad (3.8)$$

Il s'agit ici d'une contraction partielle restante($C_{pr}$), le mélange initial ayant déjà été soumis à une contraction partielle primaire ($C_{p1}$), qui se trouve en calculant la contraction totale ($C_t$) du mélange selon l'équation 1.14 ou 1.16.

### *Exemple de référence de calcul pratique selon la méthode 1b montrant l'effet des contractions partielles*

En partant d'un mélange à 85 % vol, on veut obtenir après dilution un mélange à 60 % vol et déterminer pour cela les volumes : $Vf_{mél.\,1}$ , $V_{aj\,W}$, $V_{ap\,W}$ et les contractions totales et partielles à la température de référence de 20 °C.
En appliquant la formule 3.3 et en introduisant pour $Vf_{mél.2}$ = 100 l, et 85,0 % vol ($x_1$ : 79,40 % mas) et 60 % vol ($x_2$ : 52,09 % mas et $\rho_{mél.2}$ = 909,11 kg/m$^3$) et $\rho_W$ = 998,20 kg /m$^3$. On obtient ainsi le volume ajouté d'eau suivant:

$$V_{aj\,W} = 31,326\ l$$

De là on tire selon 3.6 le volume apparent d'eau

$$V_{ap\,W} = 29,412\ l$$

Et on obtient la contraction partielle suivante :

$$C_{pr} = 31,326\ l - 29,412\ l = 1,914\ l$$

Puis on obtient selon l'équation 1.1 la contraction totale pour 100 l :

$$C_t = 3,634\ l$$

D'où selon l'équation 1.1 on obtient la contraction primaire pour 100 l:

$$C_{p1} = 1,720\ l$$

La table 4 et la figure 4 montrent très bien comment on peut se représenter une dilution selon la méthode 1b, avec un volume donné final fixe de 100 l. On peut noter que la différence entre [Vf mél. 1 + Vaj W ] et Vfmél.2 est égale directement à la contraction partielle (Cpr), et que la courbe de cette contraction a une certaine ressemblance avec celle de la contraction totale représentée dans la figure 5.

### **La contraction partielle lors de la dilution de mélanges alcool- eau ou d'eaux-de-vie par l'addition d'un autre mélange alcool-eau**

La dilution de mélanges alcool-eau ou eaux-de-vie avec un autre mélange alcool-eau de degré inférieur, est aussi une méthode de dilution couramment utilisée dans la pratique. Pour ce type de dilution, de manière analogue à la partie précédente, l'on pourra avoir : soit selon *méthode 2a* un volume déterminé fixe pour le mélange initial ($Vf_{mél.\,1}$ ); soit selon *méthode 2b* un volume déterminé fixe pour le mélange final ($Vf_{mél.\,2}$).

**Méthode 2a : Dilution par addition d'un mélange alcool-eau en partant d'un volume déterminé fixe de mélange initial ($Vf_{mél. 1}$)**

**Calcul du volume d'un mélange alcool-eau donné à rajouter à un volume de mélange alcool-eau déterminé pour obtenir un mélange final d'une teneur en alcool déterminée**

Dans la pratique de l'alcoométrie on est parfois appelé à calculer le nombre de litres d'un mélange alcool-eau d'une teneur en alcool $x_2$ ($Vf_{mél. 2}$) à ajouter à un volume donné d'un alcool ou une eau-de-vie ($Vf_{mél. 1}$) d'une teneur en alcool $x_1$ pour obtenir un autre mélange d'une teneur en alcool finale $x_3$ ( où $x_1$, $x_2$ et $x_3$ sont exprimés en % mas).

La méthode de calcul est d'abord expliquée sur la base de formules, puis démontré ensuite en pratique à l'aide d'un exemple et d'un graphique (cf. fig. 6) dans un prochain paragraphe grâce à des exemples.

Pour cela on se base sur les formules générales suivantes aussi utilisées dans l'alcoométrie traditionnelle avec la méthode dite "de la croix" (24).

$$m_{mél.3} = m_{mél.1} + m_{mél.2} \qquad (4.1)$$

$$m_{mél 3} \cdot x_3 = m_{mél.1} \cdot x_1 + m_{mél.2} \cdot x_2 \qquad (4.2)$$

De la formule 4.1 et de l'égalité [ p = masse / volume ] on obtient :

$$Vf_{mél 3} \cdot \rho_{mél.3} \ = \ Vf_{mél.1} \cdot \rho_{mél.1} + \ Vf_{mél2} \cdot \rho_{mél.2} \qquad (4.3)$$
masse du mélange 3      masse du mélange 1    masse du mélange 2

Avec ce mode de dilution selon la méthode 2a nous faisons ensuite appel aux formules suivantes développées à partir de 4.1 et 4.2 et permettant de calculer le volume $V_{aj\ mél.2}$ qui doit être ajouté au volume final $Vf_{mél. 1}$

$$m_{mél.2} = m_{mél 1} \cdot (x_1 - x_3)/(x_3 - x_2) \qquad (4.4)$$

$$V_{aj\ mél.2} \cdot \rho_{mél 2} = Vf_{mél.1} \cdot \rho_{mél.1} \cdot (x_1 - x_3)/(x_3 - x_2) \qquad (4.5)$$

$$V_{aj\ mél\ 2} = \frac{Vf_{mél.1} - \rho_{mél.1} \cdot (x_1 - x_3)/(x_3 - x_2)}{\rho_{mél.2}} \qquad (4.6)$$

Il est aussi nécessaire de calculer $Vf_{mél\ 3}$ à partir de $Vf_{mél.1}$. Avec $Vf_{mél.3}$ nous obtenons ainsi le volume du mélange alcool-eau qui tient compte de la contraction.

Pour le calcul du volume de $Vf_{mél.3}$ on peut donc procéder de même :

$$m_{mél.3} = M_{mél.1} \cdot (x_1 - x_2)/(x_3 - x_2) \qquad (4.7)$$

D'où l'on déduit logiquement:

$$Vf_{mél.3} \cdot \rho_{mél.3} = Vf_{mél.1} \cdot \rho_{mél.1} \cdot (x_1 - x_2)/(x_3 - x_2) \qquad (4.8)$$

$$Vf_{mél.3} = \frac{Vf_{mél\ 1} \cdot \rho_{mél\ 1} \cdot (x_1 - x_2)/(x_3 - x_2)}{\rho_{mél.3}} \qquad (4.9)$$

D'un autre côté on aura aussi :

$$V_{aj\ mél\ 3} = Vf_{mél.1} + V_{aj\ mél\ 2} \qquad (4.10)$$

**Calcul des contractions partielles lors de la dilution selon la méthode 2a**

Dans ce type de dilution la contraction totale est la somme de trois contractions partielles successives suivantes :

$$C_t' = C_{p1}' + C_{p2}' + C_{pr}' \qquad (4.11)$$

Dans le cadre de la méthode 2a, nous avons d'abord le volume $Vf_{mél\ 1}$ donné (=volume apparent), fixé par exemple à 100 l. Il est ainsi possible de calculer avec la formule 1.14 la contraction partielle $C_{p1}'$ de ce mélange. Nous obtenons ensuite calculé avec la formule 4.6 le volume $V_{aj\ mél.2}$ (=volume ajouté). Il est ainsi aussi possible en introduisant cette valeur dans la formule 1.14 de calculer la contraction partielle $C_{p2}'$ de ce mélange.
Enfin en ce qui concerne $C_{pr}$ on pourra procéder ainsi :

$$C_{pr}' = V_{aj\ mél.\ 3} - Vf_{mél.3} \qquad (4.12)$$

Où

$$V_{aj\ mél\ 3} = V_{aj\ mél\ 2} + Vf_{mél\ 1} \qquad (4.13)$$

Et ainsi

$$C_{pr}' = V_{aj\ mél.\ 2} + Vf_{mél.\ 1} - Vf_{mél.3} \qquad (4.14)$$

Comme déjà remarqué pour la méthode 1a les contractions ainsi obtenues sont exprimées par rapport à des volumes $Vf_{mél.3}$ qui diffèrent des 100 l pris comme référence et doivent donc être normalisée pour être comparables avec les contractions totales aussi normalisées (Ct)(cf. fig. 5 et 6).

**Exemple de calcul pratique de dilution selon la méthode 2a en tenant compte des contractions partielles**

En partant de 100 l d'un mélange à 85 % vol, on veut obtenir un mélange à 40 vol par adjonction d'un mélange à 20 % vol et déterminer pour cela les volumes : $V_{aj\ mél\ 2}$, $Vf_{mél\ 3}$ et les contractions totales et partielles à 20 °C.
On a d'abord les solutions alcooliques avec 85,0 % vol ($x_1$ : 79,40 % mas, $\rho_{mél.1}$ : 844,85 kg /m³) et avec 20 % vol ($x_2$ : 16,21 % mas, $\rho_{mél.2}$ : 973,56 kg /m³) et on veut obtenir une solution à 40 % vol ($x_3$: 33,3 % mas, $\rho_{mél.\ 3}$ : 948,05 kg /m³).
En appliquant la formule 4.6 et en posant $Vf_{mél.1}$ = 100 l on obtient :

$$V_{aj\ mél.\ 2} = 234,086\ l$$

Puis de là avec 4.9 on obtient le volume final du mélange 3 :

$$Vf_{mél.3} = 329,5\ l$$

Avec la formule 4.14 on trouve ainsi:

$$C_{pr}' = 4,586\ l\ \text{pour } 329,5\ l\ (Vf_{mél.3})$$

Et ensuite normalisé à 100 l de volume final cela donne:

$$C_{pr} = 1{,}392 \ l \ \text{pour} \ 100 \ l$$

Avec ces valeurs pour $Vf_{mél.1}$ et $V_{aj \ mél \ 2}$ et avec la formule 1.14 on obtient ensuite respectivement les contractions partielles $C_{p1}$ et $C_{p2}$:

Calculé comme $C_t$ pour 100 l ($Vf_{mél.1}$) et ramené à 329,5 l ($Vf_{mél.3}$) :

$$C_{p1}' = 2{,}435 \ l$$

Et ensuite :

$$C_{p1} = 0{,}739 \ l \ \text{pour} \ 100 \ l$$

De manière anatogue :

$$C_{p2}' = 4{,}030 \ l \ \text{pour} \ 234{,}086 \ l \ (V_{aj \ mél.2})$$

D'où:

$$C_{p2} = 1{,}223 \ l \ \text{pour} \ 100 \ l$$

De là en additionnant $C_{p1}$, $C_{p2}$ et $C_{pr}$ selon 4.11 on obtiendra la contraction totale suivante pour cet exemple de mélange à 40 % vol et 20 °C :

$$C_t' = 11{,}051 \ l \ \text{pour} \ 329{,}5 \ l \ (Vf_{mél \ 3})$$

Et de là normalisé pour 100 l:

$$C_t = 3{,}354 \ l$$

- Avec le graphique (cf. fig. 6) on peut mieux se représenter la superposition complexe de ces différentes contractions partielles. Si on veut faire des dilutions par l'addition de deux mélanges alcool-eau de 85 % vol et 20 % vol, on peut tracer deux droites.

- La première droite allant de $C_t$ à 85 % vol à la valeur de 20 % vol l'axe des x, et délimitant dans la partie inférieure les $C_{p1}$ et dans la partie supérieure les $C_{p2}$.

- La deuxième droite allant de $C_t$ :85 % vol à $C_t$ : 20 % vol délimitant dans la partie inférieure les $C_{p2}$ et dans la partie supérieure les $C_{pr}$. Avec la même méthode on peut aussi examiner une dilution à 40 ou 60 % vol et voir comment se déplacent les contractions partielles respectives.

Une grande similitude peut être observée entre la figure 5 et la figure 6, la principale différence étant cependant que dans figure 5 la droite passe par l'origine et délimite la partie supérieure les $C_{pr}$ et dans la partie inférieure $C_{p1}$.

**Méthode 2b: Dilution par addition d'un mélange alcool-eau pour obtenir un volume déterminé fixe du mélange final ($Vf_{mél. 2}$)**

**Calcul des volumes d'un mélange alcool-eau (1) et d'un mélange alcool-eau (2), de degré inférieur, à ajouter l'un à l'autre pour obtenir un mélange final d'un volume et d'une teneur en alcool déterminés.**

Par analogie à la méthode 1b, on se propose de calculer ici le nombre de litres d'un mélange alcool-eau d'une teneur en alcool en % vol [ $x_1$ ]($Vf_{mél\ 1}$) et le nombre de litres d'un alcool ou une eau-de-vie ($Vf_{mél.\ 2}$) d'une teneur en alcool % vol [ $x_2$ ] à ajouter, pour obtenir un mélange d'une teneur en vol % [$x_3$] et d'un volume exactement déterminés en tenant bien compte de la contraction. Ici aussi la méthode de calcul est d'abord expliquée sur la base de formules, puis démontré ensuite en pratique grâce à des exemples.

Pour cela on se base également sur les formules générales 4.1, 4.2 et 4.3 aussi utilisées dans l'alcoométrie traditionnelle avec la méthode dite "de la croix"(24).

Dans le contexte du mode de dilution choisi nous faisons ensuite appel aux formules suivantes développées à partir de 4.1 et 4. 2 et permettant de calculer les volumes $V_{aj\ mél.\ 1}$ et $V_{aj\ mél.2}$ qui doivent être ajoutés pour obtenir un le volume $Vf_{mél.3}$ fixé par exemple à 100 l, avec une teneur en alcool déterminée.

$$m_{mél.\ 1} = m_{mél.\ 3} \cdot (x_3 - x_2)/(x_1 - x_2) \tag{5.1}$$

$$V_{aj\ mél.\ 1} \cdot \rho_{mél.1} = Vf_{mél.3} \cdot \rho_{mél\ 3} \cdot (x_3 - x_2)/(x_1 - X_2) \tag{5.2}$$

$$V_{aj\ mél.\ 1} = \frac{Vf_{mél\ 3} \cdot \rho_{mél.3} \cdot (x_3 - x_2)/(x_1 - x_2)}{\rho_{mél\ 1}} \tag{5.3}$$

Pour le calcul du volume de $V_{aj\ mél\ 2}$ on procède de la manière analogue:

$$m_{mél.\ 2} = m_{mél\ 3} \cdot (x_1 - x_3)/(x_1 - x_2) \tag{5.4}$$

$$V_{aj\ mél\ 2} \cdot \rho_{mél.2} = Vf_{mél.\ 3} \cdot \rho_{mél\ 3} \cdot (x_1 - x_3)/(x_1 - x_2) \tag{5.5}$$

$$V_{aj\ mél.2} = \frac{Vf_{mél.3} \cdot \rho_{mél.3} \cdot (x_1 - x_3)/(x_1 - x_2)}{\rho_{mél..2}} \tag{5.6}$$

N.B. : Il est aussi à noter que une des difficulté de tels calculs vient du fait que l'on fixe d'abord un volume final apparent, ici $Vf_{mél.3}$ , et que l'on recherche ensuite les volume ajoutés, ici $V_{aj\ mél.\ 1}$, $V_{aj\ mél\ 2}$ , dont la somme est supérieure au $Vf_{mél.3}$.

Finalement on aura donc:

$$V_{aj\ mél.3} = V_{aj\ mél\ 1} + V_{aj\ mél\ 2} \tag{5.7}$$

Et ainsi :

$$C_{pr} = V_{aj\ mél.\ 3} - Vf_{mél\ 3} \tag{5.8}$$

Alors que $C_{p1}$ et $C_{p2}$ peuvent se calculer directement à partir des formules 1.14 et 1.16, l'on obtient $C_t$ en additionnant $C_{p1.}$ $C_{p2}$ et $C_{pr}$ (cf. 4.11).

**Exemple pratique : préparation selon la méthode 2b d'un mélange final recherché en déterminant les mélange alcooliques a et b à ajouter compte tenu des contractions**

En prenant le même exemple que dans le paragraphe précédent et appliquant les formules 5.3 et 5.6 en posant $Vf_{mél.3}$ = 100 l on obtient les volumes des mélanges 2 et 3 suivants :

$$V_{aj\ mél\ 1} = 30.349\ l$$

Et puis :

$$V_{aj\ mél.2} = 71,043\ l$$

Avec 5.7 et 5.8 on trouve ainsi directement :

$$C_{pr} = 1,392\ l\ \text{pour 100 l}$$

Avec ces valeurs pour $V_{aj\ mél.\ 1}$ et $V_{aj\ mél\ 2}$ et avec la formule 1. 14 et 1. 16 on obtient ensuite les contractions partielles suivantes :

$$C_{t}'_{(Cp1)} = 0,739\ l\ \text{pour}\ V_{aj\ mél.\ 1} = 30.349\ l$$

D'où reporté à 100 l de volume final, on a ainsi directement la valeur $C_{p1}$:

$$C_{p1} = 0,\ 739\ l\ (\text{pour}\ Vf_{mél\ 3} = 100\ l\ )$$

De manière analogue :

$$C_{t}'_{(Cp2)} = 1,223\ l\ \text{pour}\ V_{aj\ mél.2} = 71,043\ l$$

Et ce qui reporté à 100 l de mélange final donne aussi directement:

$$C_{p2} = 1,223\ l\ (\text{pour}\ Vf_{mél\ 3} = 100\ l\ )$$

De là en additionnant $C_{p1}$, $C_{p2}$ et $C_{pr}$ (cf. 4.11) on obtiendra directement la contraction totale pour cet exemple de mélange à 40 % vol et 20 °C :

$$C_{t} = 3,354\ l\ \text{pour 100 l}$$

**En conclusion:**

Il peut être remarqué que les valeurs obtenues avec la méthode 2a coïncident bien à ceux obtenus avec la méthode 2b, et s'intègrent aussi bien dans le graphique calculé (cf. fig. 5). Ceci est précieux et tend à prouver que les résultats obtenus aussi dans cette situation compliquée sont cohérents, alors qu'ils ont été calculés sur la base de données utilisées de manière tout à fait différente. De même une bonne cohérence est relever entre les résultats obtenus méthodes 1a et 2a. D'autre part, comme montré par les figures 5 et 6, les contractions totales et partielles trouvées pour respectivement les méthodes 1a, 1b, 2a et 2b sont en bon accord.

En se basant sur les connaissances assez fragmentaires à disposition il a été essayé d'effectuer ici une étude plus systématique et détaillée de la contraction et en particulier de la contraction partielle dans les mélanges éthanol-eau. Les résultats présentés ici ont vraisemblablement ouvert de nouveaux horizons sur un domaine particulièrement complexe. La meilleure connaissance de ces effets et des calculs, qui permettent de les mettre en évidence, aura sûrement une utilité d'une part théorique, mais aussi pratique comme par exemple lors de la dilution des mélanges éthanol-eau.

L'application de ces différentes notions nouvelles pourra avoir en particulier des applications sur le plan industriel, pour mieux maîtriser à ce niveau la préparation de solution alcool-eau, avec des teneurs en alcool et des volumes très bien déterminés, compte tenu des températures.

D'autre part il ne semble pas y avoir de raisons que les principes développés ici pour ce mélange éthanol-eau ne soient pas valables de manière plus générale et puissent être également appliqués à d'autres alcools et solvants.

**Bibliographie**

1. *Bruun, G. S.*, *Graae Soerensen, P. and Hvidt*, *Aa.:* Ultrasonic properties of ethanol-water mixtures. Acta Chem. Scand. A28, 1047-1054 (1974).

2. *v. Goldammer*, *E and Hertz, H. G.:* Molecular motion and structure of aqueous mixtures with non-electrolytes as studied by nuclear magnetic relaxation methods. J. Phys. Chem. 74, 3734-3755 (1970).

3. *v. Goldammer*, *E. and Zeidler*, *M. D.*: Molecular motion in aqueous mixtures with organic liquids by NMR relaxation measurements. Ber. Bunsenges. Physik. Chem. 73, 4-15 (1969 ).

4. *Beddard*, *G. S.*, *Doust*, *T. and Hudales*, J.: Structural features in ethanol-water mixtures revealed by picosecond fluorescence anisotropy. Nature 294, 145-146 (1981).

5. *Beddard*, *G.S.*, *Doust, T. and Porter, G.:* Fluorescence depolarization measured by frequency conversion. Chem. Phys. 61, 17-23 (1981).

6. *Fleming, G. R., Morris, J. M. and Robinson,* G. *W.:* Direct observation of rotational diffusion by picosecond spectroscopy. Chem. Phys. 17, 91-100 (1976).

7. Viscosity, aqueous solutions, non - electrolytes,: International Critical Tables, III 1 ed., published by the National Research Council / by Mc Graw-Hill Inc, New- York, 22 - 25 (1928).

8. *Schindler*, *W.:* Rotation of ethanol in ethanol-water mixtures studied by light scattering. Chem. Phys. 31, 345-355 ( 1978 ).

9. *Denison, R. B.*: Contributions to the knowledge of liquid mixtures I and II. Trans. Faraday Soc. 8, 20 ( 1912 ) and 8, 35 ( 1912 ).

10. *Job, P.:* Ann. Chim. (Ser. 10) 9, 113 ( 1928 ).

11. *Hill*, *Z. D. et Mac Carthy, P.*: Novel approach to Job's method (an under-graduate experiment ). J. Chem. Educ. 63, 162-167, (1986).

12. *Ostromisslensky, I.* : Über eine neue, auf dem Massenwirkungsgesetz fussende Analysenmethode einiger binären Verbindungen. Berichte 44, 268 (1911).

13. *Marcantonatos, M. et Liebich, B.W.:* Système luminescent HMCB-$H_3BO_3$ -hydroxy -2-méthoxy-4-chloro-4'-benzophenone en milieu sulfurique concentré Chimia. 24, (12) (1971).

14. *Liebich, B.W.*: Etude du système luminescent HMCB-$H_3BO_3$-hydroxy-2-méthoxy-4-chloro-4'-benzophenone en milieu sulfurique conc. et ses applications au dosage de traces de bore dans les eaux, sols et plantes. Thèse Nr. 1556 Université de Genève (1971).

15. *Steiner*, *T., Mason, S. A. and Saenger*, *W.*: Cooperative O - H..O hydrogen bonds in β-cyclodextrine-ethanol octahydrate at 15° K : A neutron diffraction study. J. Am.Chem.Soc., 112, 6184-6190 (1990).

16. *Speedy*, *R. J.*: Self-replicating structures in water J. Phys. Chem. 88, 3364 - 3373 (1984).

**EP 1 112 992 B1**

17. *Plato*, *F.:* Tafel zur Umrechnung der Volumenprozente in Gewichtsprozente und der Gewichtsprozente in Volumenprozente bei Branntweinen. Verlag Julius Springer, Berlin 1901.

18. *Osborne, N. S., McKelvy*, *E. C. and Bearce, H. W.:* Density and thermal expansion of ethyl alcohol and ist mixtures with water. Bulletin of the bureau of Standards, 9, 327-474 (1913).

19. *Feydt*, *G.:* Tabellen zur Kontraktionsberechnung (erstellt nach Angaben von F. Plato). Institut für Gärungsgewerbe, Berlin (ca. 1930).

20. *Angelidis, O. M.:* Calcul du mélange de l'alcool éthylique avec de l'eau. Chimie Analytique 50, 118-121 (1968).

21. *Potterat*, *M.:* Régie féd. des alcools, publication interne: Dilution et remontage, Berne (1973).

22. Organisation internationale de métrologie légale ( OIML), Tables alcoométriques internationales, Bureau international de métrologie légal, Paris 1975.

23. Office fédéral de métrologie: Tables alcoométriques ( établies selon les recommandations de l'OIML ). EDMZ, Berne 1977.

24. Régie féd. des alcools, publication interne: Table pour le calcul du volume occupé à 20 °C, Berne (1983).

25. Régie féd. des alcools et Office fédéral de métrologie: Tables alcoométriques pratiques ( 1984 ).

26. Régie féd. des alcools, publication interne: Dilution des eaux-de-vie, Berne (1985).

27. Bundesmonopolverwaltung für Branntwein: Alkoholrechenprogramm (EDV), Offenbach / Main (1996).

28. Tables alcoométriques du PTB / Allemagne ( calculées selon les recommandations de l' OIML ), adaptées pour la Suisse, Berne (1997).

**Revendications**

1. Procédé pour la préparation d'un mélange alcool-eau d'une teneur finale donnée en mélangeant un mélange alcool-eau d'une première teneur avec un mélange alcool-eau d'une deuxième teneur à une température déterminée, et le mélange obtenu ou un mélange initial ont un volume précis donné, **caractérisé en ce que** en considérant les contractions entières ou partielles de volumes des mélanges alcool-eau, on établit un tableau avec une pluralité des pairs des valeurs dans tout le domaine jusqu'à 100 % de volume contenant la teneur en alcool en % de volume la masse volumique correspondante à une température constante ou que l'on calcule à l'aide d'une formule mathématique décrivant la teneur en alcool en fonction de la masse volumique à une température constante, pour une teneur d'alcool la masse volumique correspondante ou vice versa ou que d'autres valeurs pertinentes ayant une relation mathématique avec ces valeurs mentionnées comme % en masse ou la contraction de volume ou le volume du mélange sont calculées, et les valeurs obtenues sont utilisées pour déterminer la masse ou le volume des liquides de départ et que l'on utilise ces valeurs pour déterminer la quantité nécessaire au moins d'un liquide de départ pour la préparation du mélange alcool-eau avec la teneur finale donnée le cas échéant avec le volume prédéterminé.

2. Procédé selon la revendication 1 **caractérisé en ce que** le tableau avec la pluralité des pairs des valeurs dans tout le domaine iusqu'à 100 % de volume contenant la teneur en alcool en % de volume la masse volumique correspondante à une température constante ou la formule mathématique décrivant la teneur en alcool en fonction de la masse volumique à une température constante sont chargées dans un système d'ordinateur qui affiche pour chaque teneur d'alcool la masse volumique correspondante ou vice versa ou qui affiche des autre valeurs pertinentes ayant une relation mathématique aux valeurs mentionnées, et les valeurs obtenues sont utilisé pour l'indication de la masse ou du volume des liquides de départ et que le système d'ordinateur utilise ces valeurs pour déterminer la quantité nécessaire au moins d'un liquide de départ pour la préparation du mélange alcool-eau avec la teneur finale donnée le cas échéant avec le volume prédéterminé; où les valeurs calculées par le system d'ordinateur sont utilisées pour contrôler des soupapes d'un système de dosage pour la préparation automatique du mélange alcool-eau prédéterminé.

26

3. Procédé selon la revendication 1 ou 2 pour la préparation d'un mélange alcool-eau d'une troisième teneur donnée en mélangeant une masse ou un volume prédéterminé d'un mélange alcool-eau d'une première teneur majeure avec un mélange alcool-eau d'une deuxième teneur inférieure à une température déterminée, et la troisième teneur soit inférieure de la première teneur et majeur à la deuxième teneur, **caractérisé en ce que** la masse ou le volume du mélange alcool-eau de la deuxième teneur sont calculés par les valeurs de la teneur du mélange alcool-eau de la troisième teneur donnée, de la masse ou du volume et de la teneur du mélange alcool-eau de la première teneur et de la teneur du mélange alcool-eau de la deuxième teneur.

4. Procédé selon la revendication 1 ou 2 pour la préparation d'un volume ou d'une masse prédéterminé d'un mélange alcool-eau d'une troisième teneur donnée en mélangeant un mélange alcool-eau d'une première teneur majeure avec un mélange alcool-eau d'une deuxième teneur inférieure à une température déterminée, et la troisième teneur soit inférieure de la première teneur et majeure de la deuxième teneur, **caractérisé en ce que** la masse ou le volume du mélange alcool-eau de la première teneur et du mélange alcool-eau de la deuxième teneur sont calculés par les valeurs du volume ou de la masse et de la teneur du mélange alcool-eau de la troisième teneur donnée, de la teneur du mélange alcool-eau de la première teneur et de la teneur du mélange alcool-eau de la deuxième teneur.

5. Procédé selon l'une des revendications 1 à 4, **caractérisé en ce que** l'on utilise pour les mélanges un alcool qui est miscible complètement avec de l'eau, par exemple du méthanol, de l'éthanol, du propanol, de l'isopropanol, du butanol ou de terbutanol.

6. Procédé selon les revendications 3 et 5, **caractérisé en ce que** l'on utilise l'équation suivante pour déterminer la masse ou le volume du mélange alcool-eau d'une deuxième teneur à ajouter:

$$V_{aj\ mél\ 2} = \frac{Vf_{mél\ 1} \cdot \rho_{mél\ 1} \cdot (x_1 - x_3)/(x_3 - x_2)}{\rho_{mél\ 2}}$$

$$Vf_{mél\ 3} = \frac{Vf_{mél\ 1} \cdot \rho_{mél\ 1} \cdot (x_1 - x_2)/(x_3 - x_2)}{\rho_{mél\ 3}}$$

$$V_{aj\ mél\ 3} = Vf_{mél\ 1} + V_{aj\ mél\ 2}$$

$$C_{pr}' = V_{aj\ mél\ 2} + Vf_{mél\ 1} - Vf_{mél\ 3}$$

$V_{aj\ mél\ 2} =$     volume du mélange d'une deuxième teneur (initial)

$V_{aj\ mél\ 3} =$     volume du mélange d'une troisième teneur (initial)

$Vf_{mél\ 1} =$     volume final prédéterminé du premier mélange d'une première teneur

$Vf_{mél\ 3} =$     volume final du troisième mélange d'une troisième teneur

$\rho_{mél\ 1} =$     masse volumique du premier mélange d'une première teneur (initial)

$\rho_{mél\ 2} =$     masse volumique du deuxième mélange d'une deuxième teneur (initial)

$\rho_{mél\ 3} =$     masse volumique du troisième mélange d'une troisième teneur (initial)

$x_1 =$     teneur en alcool (% mas) du mélange d'une première teneur (initial)

$x_2 =$     teneur en alcool (% mas) du mélange d'une deuxième teneur (initial)

$x_3 =$     teneur en alcool (% mas) du mélange d'une troisième teneur (final)

$C_{pr}'$ = contraction partielle restante (non normalisée)

7. Procédé selon les revendications 4 et 5, **caractérisé en ce que** l'on utilise les équations suivantes pour déterminer la masse ou le volume du mélange alcool-eau de la première teneur et du mélange alcool-eau de la deuxième teneur à ajouter:

$$V_{aj\ mél\ 1} = \frac{Vf_{mél\ 3} \cdot \rho_{mél\ 3} \cdot (x_3 - x_2)/(x_1 - x_2)}{\rho_{mél\ 1}}$$

$$V_{aj\ mél\ 2} = \frac{Vf_{mél\ 3} \cdot \rho_{mél\ 3} \cdot (x_1 - x_3)/(x_1 - x_2)}{\rho_{mél\ 2}}$$

$$V_{aj\ mél\ 3} = V_{aj\ mél\ 1} + V_{aj\ mél\ 2}$$

$$C_{pr} = V_{aj\ mél\ 3} - Vf_{mél\ 3}$$

$V_{aj\ mél\ 1}$ = volume du mélange d'une premier teneur (initial)

$V_{aj\ mél\ 2}$ = volume du mélange d'une deuxième teneur (initial)

$V_{aj\ mél\ 3}$ = volume du mélange d'une troisième teneur (initial)

$Vf_{mél\ 3}$ = volume final prédéterminé du troisième mélange d'une troisième teneur

$\rho_{mél\ 1}$ = masse volumique du mélange d'une première teneur (initial)

$\rho_{mél\ 2}$ = masse volumique du troisième mélange d'une deuxième teneur (initial)

$\rho_{mél\ 3}$ = masse volumique du troisième mélange d'une troisième teneur (initial)

$x_1$ = teneur en alcool (% mas) du mélange d'une première teneur (initial)

$x_2$ = teneur en alcool (% mas) du mélange d'une deuxième teneur (initial)

$x_3$ = teneur en alcool (% mas) du mélange d'une troisième teneur (final)

$C_{pr}$ = contraction partielle restante (normalisée si $Vf_{mél\ 3}$ = 100)

8. Dispositif pour accomplir le procédé selon l'une des revendications 1 à 7, **caractérisé en ce qu'**il comprend les éléments suivants:

- au moins deux enceintes pour accueillir les liquides de départ contenant des mélanges eau-alcool,
- une enceinte de mélange pour accueillir les liquides à mélanger pour la préparation du mélange, comprenant le cas échéant un moyen pour agiter,
- un système de tuyaux et de soupapes, avec lesquelles les enceintes pour accueillir les liquides de départ sont liées avec l'enceinte de mélange dans une manière que des quantités quelconques des liquides contenues peuvent être transférées dans l'enceinte de mélange, en réglant les soupapes
- un système d'ordinateur de réglage pour les soupapes, le système d'ordinateur étant programmé d'une tel manière qu'il règle ces soupapes à base d'un tableau avec une pluralité de valeurs comprenant les % en volume et la masse volumique correspondante dans la domaine de 0 à 100 % vol teneur d'alcool d'une température constante chargé dans l'ordinateur ou à base d'une fonction mathématique qui décrit la teneur d'alcool en fonction de la masse volumique à une température constante pour que les volumes corrects soient transférés dans l'enceinte de mélange pour obtenir un mélange avec le volume et la teneur d'alcool cherchée.

**Patentansprüche**

1. Verfahren zur Herstellung einer Alkohol- Wasser Mischung mit einem vorbestimmtem Endgehalt, indem eine Alkohol-Wasser Mischung eines ersten Gehaltes mit einer Alkohol-Wasser Mischung eines zweiten Gehaltes bei einer vorbestimmten Temperatur gemischt wird, und die erhaltene Mischung oder eine Ausgangsmischung ein genau vorbestimmtes Volumen haben, **dadurch gekennzeichnet, dass** man unter Berücksichtigung der totalen oder partiellen Volumenkontraktionen der Alkohol-Wasser Mischungen eine Tabelle mit einer Vielzahl von Wertepaaren im ganzen Bereich bis 100 %vol erstellt, enthaltend den Alkoholgehalt in %vol, die entsprechende Dichte bei einer konstanten Temperatur, oder dass mit Hilfe einer mathematischen Formel, die den Alkoholgehalt als Funktion der Dichte bei einer konstanten Temperatur beschreibt, für einen Alkoholgehalt die entsprechende Dichte oder umgekehrt berechnet wird, oder dass andere sachdienliche Werte, die in einer mathematischen Beziehung zu den genannten Werten, wie %mas oder der Volumenkontraktion, oder Volumen der Flüssigkeit, stehen, berechnet werden, und die erhaltenen Werte dazu verwendet werden um die Masse oder das Volumen der Ausgangsflüssigkeiten zu bestimmen, und dass diese Werte verwendet werden um die erforderliche Menge einer Ausgangsflüssigkeit für die Herstellung der Alkohol-Wasser Mischung mit einem bestimmten Gehalt und gegebenenfalls mit vorbestimmtem Volumen zu bestimmen.

2. Verfahren gemäss Anspruch 1, **dadurch gekennzeichnet, dass** die Tabelle mit der Vielzahl von Wertepaare im gesamten Bereich bis 100 %vol, die den Alkoholgehalt in %vol, die entsprechende Dichte bei einer konstanten Temperatur umfasst, oder die mathematische Formel, welche den Alkoholgehalt als Funktion der Dichte bei einer konstanten Temperatur beschreibt, in ein Computersystem geladen werden, welches für jeden Alkoholgehalt die entsprechende Dichte oder umgekehrt anzeigt, oder andere sachdienliche Werte, die in mathematischer Beziehung zu den erwähnten Werten stehen, anzeigt, und die erhaltenen Werte zum Anzeigen der Masse oder des Volumens mindestens einer Ausgangsflüssigkeit für die Herstellung einer Alkohol-Wasser-Mischung mit einem vorgegebenen Endgehalt, gegebenenfalls mit vorbestimmtem Volumen dienen; oder die mit Computersystem berechneten Werte verwendet werden, um die Ventile eines Dosiersystems zur automatischen Herstellung einer vorgegebenen Alkohol Wasser Mischung zu steuern.

3. Verfahren gemäss Anspruch 1 oder 2 für die Herstellung einer Alkohol-Wasser Mischung eines dritten vorgegeben Gehaltes durch Mischen einer Masse oder eines Volumen einer Alkohol-Wasser Mischung eines ersten höheren Gehaltes mit einer Alkohol-Wasser Mischung eines zweiten tieferen Gehalt bei einer vorbestimmten Temperatur, und der dritte Gehalt tiefer als der erste Gehalt und höher ist als der zweite Gehalt, **dadurch gekennzeichnet, dass** die Masse oder das Volumen der Alkohol-Wasser Mischung mit dem zweiten Gehaltes aus den Werten des vorgegebenem dritten Gehaltes, aus der Masse oder dem Volumen und dem Gehalt der Alkohol-Wasser Mischung des ersten Gehaltes und dem Gehalt der Alkohol-Wasser Mischung des zweiten Gehaltes berechnet werden.

4. Verfahren gemäss Anspruch 1 oder 2 für die Herstellung eines vorbestimmten Volumens oder Masse einer Alkohol-Wasser Mischung eines dritten vorgegeben Gehaltes, indem eine Masse oder ein Volumen einer Alkohol-Wasser Mischung eines ersten höheren Gehaltes mit einer Alkohol-Wasser Mischung eines zweiten tieferen Gehalt bei einer gegebenen Temperatur gemischt werden, und der dritte Gehalt tiefer als der erste Gehalt und höher als der zweite Gehalt ist, **dadurch gekennzeichnet dass** die Masse oder das Volumen der Alkohol-Wasser Mischung des ersten Gehaltes und der Alkohol-Wasser Mischung des zweiten Gehaltes aus den Werten der Masse oder der Volumens und aus dem vorgegebenem dritten Gehalt, aus dem Gehalt der Alkohol-Wasser Mischung des ersten Gehaltes und dem Gehalt der Alkohol-Wasser Mischung des zweiten Gehaltes berechnet werden.

5. Verfahren gemäss Anspruch 1 bis 4, **dadurch gekennzeichnet, dass** für die Mischungen ein Alkohol verwendet wird, der vollständig mit Wasser mischbar ist, wie zum Beispiel Methanol, Ethanol, Propanol, Isopropanol, Butanol oder ter-Butanol.

6. Verfahren gemäss den Ansprüchen 3 und 5, **dadurch gekennzeichnet, dass** man die folgenden Formeln verwendet um die Masse oder das Volumen der Alkohol-Wasser Mischung eines zweiten Gehaltes, die zugegeben wird, zu ermitteln:

$$V_{aj\,m\acute{e}l\,2} = \frac{Vf_{m\acute{e}l\,1} \cdot \rho_{m\acute{e}l\,1} \cdot (x_1 - x_3)/(x_3 - x_2)}{\rho_{m\acute{e}l\,2}}$$

$$Vf_{mél\ 3} = \frac{Vf_{mél\ 1} \cdot \rho_{mél\ 1} \cdot (x_1 - x_2)/(x_3 - x_2)}{\rho_{mél\ 3}}$$

$$V_{aj\ mél\ 3} = Vf_{mél\ 1} + V_{aj\ mél\ 2}$$

$$C_{pr}' = V_{aj\ mél\ 2} + Vf_{mél\ 1} - Vf_{mél\ 3}$$

$V_{aj\ mél\ 2}$ = Volumen der Mischung mit einem zweiten Gehalt (anfänglich)

$V_{aj\ mél\ 3}$ = Volumen der Mischung mit einem dritten Gehalt (anfänglich)

$Vf_{mél\ 1}$ = Vorbestimmtes Volumen der Mischung mit einem ersten Gehalt (anfänglich)

$Vf_{mél\ 3}$ = Volumen der Mischung mit einem dritten Gehalt (engültig)

$\rho_{mél\ 1}$ = Dichte der Mischung mit einem ersten Gehalt (anfänglich)

$\rho_{mél\ 3}$ = Dichte der Mischung mit einem dritten Gehalts (endgültig)

$x_1$ = Alkoholgehalt (%mas) der Mischung mit einem ersten Gehalt (anfänglich)

$X_2$ = Alkoholgehalt (%mas) der Mischung mit einem zweiten Gehalt (anfänglich)

$X_3$ = Alkoholgehalt (%mas) der Mischung mit einem dritten Gehalt (am Ende).

$C_{pr}'$ = bleibende partielle Kontraktion (nicht normalisiert)

7. Verfahren gemäss den Ansprüchen 4 und 5, **dadurch gekennzeichnet, dass** man die folgenden Formeln verwendet um die Masse oder das Volumen der Alkohol- Wasser Mischung eines ersten Gehaltes und die Masse oder das Volumen der Alkohol- Wasser Mischung eines zweiten Gehaltes, die zugeben werden, zu ermitteln:

$$V_{aj\ mél\ 1} = \frac{Vf_{mél.3} \cdot \rho_{mél\ 3} \cdot (x_3 - x_2)/(x_1 - x_2)}{\rho_{mél\ 1}}$$

$$V_{aj\ mél\ 2} = \frac{Vf_{mél\ 3} \cdot \rho_{mél\ 3} \cdot (x_1 - x_3)/(x_1 - x_2)}{\rho_{mél\ 2}}$$

$$V_{aj\ mél\ 3} = V_{aj\ mél\ 1} + V_{aj\ mél\ 2}$$

$$C_{pr} = V_{aj\ mél\ 3} - Vf_{mél\ 3}$$

$V_{aj\ mél\ 1}$ = Volumen der Mischung mit einem ersten Gehalt (anfänglich)

$V_{aj\ mél\ 2}$ = Volumen der Mischung mit einem zweiten Gehalt (anfänglich)

$V_{aj\ mél\ 3}$ = Volumen der Mischung mit einem dritten Gehalt (anfänglich)

$Vf_{mél\ 3}$ = Vorbestimmtes finales Volumen der Mischung mit einem dritten Gehalt

$\rho_{mél\ 1}$ = Dichte einer Mischung mit einem ersten Gehalt (anfänglich)

$\rho_{\text{mél }2} =$      Dichte einer Mischung mit einem zweiten Gehalt (anfänglich)

$\rho_{\text{mél }3} =$      Dichte einer Mischung mit einem dritten Gehalt (anfänglich)

$x_1 =$      Alkoholgehalt (%mas) der Mischung mit einem ersten Gehalt (anfänglich)

$X_2 =$      Alkoholgehalt (%mas) der Mischung mit einem zweiten Gehalt (anfänglich)

$X_3 =$      Alkoholgehalt (%mas) der Mischung mit einem dritten Gehalt (anfänglich)

$C_{\text{pr}} =$      partielle restliche Kontraktion (normalisiert, wenn $Vf_{\text{mél }3} = 100$)

8. Vorrichtung zur Durchführung des Verfahrens gemäss einem der Ansprüchen 1 bis 7 **dadurch gekennzeichnet dass** es folgende Elemente enthält:

- mindestens zwei Behälter zur Aufnahme der Ausgangsflüssigkeiten umfassend Alkohol-Wasser Mischungen,

- einen Mischbehälter zur Aufnahme der zu mischenden Flüssigkeiten, der gegebenenfalls mit einem Rührsystem versehen ist,

- ein System von Rohren und Ventilen mit welchen die Behälter zur Aufnahme der Ausgangsflüssigkeiten und der Mischbehälter in solcher Weise verbunden sind, damit beliebige Mengen der enthaltenen Flüssigkeiten durch Einstellung der Ventile in den Mischbehälter geleitet werden können,

- ein Computersystem zur Steuerung der Ventile, wobei das Computersystem in solcher Weise programmiert ist, dass es die Ventile steuert, basierend auf einer Tabelle die eine Mehrzahl von Werten enthält, die in den Computer eingegeben werden, wie %vol und die entsprechende Dichte im Bereich bis 100 % bei konstanter Temperatur, oder gestützt auf eine mathematische Funktion, die den Alkoholgehalt als Funktion der Dichte bei konstanter Temperatur beschreibt, damit die korrekten Volumen in den Mischbehälter geleitet werden um eine Mischung mit dem genau erwünschten Volumen und Alkoholgehalt zu erhalten.

## Claims

1. A process for preparing of an alcohol-water mixture with a predetermined final content obtained by mixing an alcohol-water mixture of a first content with an alcohol-water mixture of a second content at a fixed temperature, and where the obtained mixture or an initial mixture have an exactly determined volume, **characterized in that** under consideration of the total or partial contractions of the volumes of the alcohol-water mixtures, a table with a plurality of pairs of values in the whole range up to 100 %vol is established, comprising the alcohol content in %vol, the corresponding density at constant temperature, or that with the help of a mathematical formula, giving the alcohol content as a function of the density at constant temperature for an alcohol content the corresponding density is calculated or vice versa, or that other pertinent values standing in a mathematical relation to the previous values such as the %mas, the volume contraction or the volume of the liquid are calculated, and that the obtained values are used to determine the mass or the volume of the starting liquids, and that these values are used to calculate the necessary quantity of at least one of the initial liquids for the preparation of the final alcohol-water mixture with a given final content and optionally with a predetermined volume.

2. The process according to claim1, **characterized in that** the table with the plurality of pairs of values within the entire range up to 100 % vol containing the alcohol content in %vol, the corresponding density at a constant temperature, or the mathematical formula describing the alcohol content as function of the density at a constant temperature, are loaded into a computer system which diaplays for each alcohol content the corresponding density or vice versa or which displays other pertinent values having a mathematical relation to the other mentioned values, and the obtained values are used to display the mass or the volume of the starting liquids and that the computer system uses these values to determine the quantity of at least one of the departure liquids necessary for the preparation of the alcohol-water mixture with a given final content and optionally with a predetermined volume; where the values calculated with the computer system are used, in order to control the valves of a dosing system for the automatic preparation of the predetermined alcohol-water mixture.

3. The process according to claim 1 or 2 for the preparation of an alcohol-water mixture of a third given content obtained by mixing a mass or a predetermined volume of an alcohol-water mixture of a first higher content with an alcohol-water mixture of a second deeper content at a predetermined temperature, and the third content being deeper than the first content and higher than the second content, **characterised in that** values of the mass or the volume of the alcohol-water mixture with second content are calculated from the values of the third given content, or from the volume and the content of the alcohol-water mixture of the first content and the content of the alcohol-water mixture of the second content.

4. The process according to claim 1 or 2 for the preparation of a predetermined volume or mass of an alcohol-water mixture of a third content, obtained by mixing a mass or a volume of an alcohol-water of a first higher content with an alcohol-water a mixture of a second deeper content at a given temperature, and where the third content is deeper than the first content and higher than the second content, **characterized in that** the mass or the volume of the alcohol-water mixture of the first content and the alcohol-water mixture of the second content are calculated from the values of the mass or of the volume and the predetermined third content and the content of an alcohol-water mixture of a second content.

5. The process according to claim 1 or 4, **characterized in that** for the mixtures an alcohol is used, which is completely miscible with water as for example methanol, ethanol, 1-propanol, 2-propanol, butanol or ter-butanol.

6. The process according to the claims 3 and 5, **characterized in that** the following formulas are used to determine the mass or the volume of the alcohol-water mixture of a second content, which is added:

$$V_{aj\ mél\ 2} = \frac{Vf_{mél\ 1} \cdot \rho_{mél\ 1} \cdot (x_1 - x_3)/(x_3 - x_2)}{\rho_{mél\ 2}}$$

$$Vf_{mél\ 3} = \frac{Vf_{mél\ 1} \cdot \rho_{mél\ 1} \cdot (x_1 - x_2)/(x_3 - x_2)}{\rho_{mél\ 3}}$$

$$V_{aj\ mél\ 3} = Vf_{mél\ 1} + V_{aj\ mél\ 2}$$

$$C_{pr}' = V_{aj\ mél\ 2} + Vf_{mél\ 1} - Vf_{mél\ 3}$$

$V_{aj\ mél\ 2} =$ volume of the mixture of a second content (initial)

$V_{aj\ mél\ 3} =$ volume of the mixture of a third content (initial)

$Vf_{mél\ 1} =$ final volume of the mixture of a first content

$Vf_{mél.3} =$ final volume of the mixture of a third content

$\rho_{mél\ 1} =$ density of a mixture of a first content (initial)

$\rho_{mél\ 2} =$ density of a mixture of a second content (initial)

$\rho_{mél\ 3} =$ density of a mixture of a third content (final)

$x_1 =$ alcohol content (% mas) of the mixture of a first content (initial)

$x_2 =$ alcohol content (% mas) of the mixture of a second content (initial)

$x_3 =$ alcohol content (% mas) of the mixture of a third content (final)

$C_{pr}' =$ partial remaining contraction (not normalised)

7. The process according to claims 4 and 5, **characterized in that** the following formulas are used to determine the mass or the volume of the alcohol-water mixture of a first content and the mass or the volume of the alcohol-water mixture of a second content, which is added:

$$V_{aj\,mél\,1} = \frac{Vf_{mél\,3} \cdot \rho_{mél\,3} \cdot (x_3 - x_2)/(x_1 - x_2)}{\rho_{mél\,1}}$$

$$V_{aj\,mél\,2} = \frac{Vf_{mél\,3} \cdot \rho_{mél\,3} \cdot (x_1 - x_3)/(x_1 - x_2)}{\rho_{mél\,2}}$$

$$V_{aj\,mél\,3} = V_{aj\,mél\,1} + V_{aj\,mél\,2}$$

$$C_{pr} = V_{aj\,mél\,3} - Vf_{mél\,3}$$

$V_{aj\,mél\,1} =$     volume of the mixture of a first content (initial)

$V_{aj\,mél\,2} =$     volume of the mixture of a second content (initial)

$Vf_{mél\,3} =$     predetermined volume of the mixture of a third content (final)

$\rho_{mél\,1} =$     density of a mixture of a first content (initial)

$\rho_{mél\,2} =$     density of a mixture of a second content (initial)

$\rho_{mél\,3} =$     density of a mixture of a third content (final)

$x_1 =$     alcohol content (% mas) of the mixture of a first content (initial)

$x_2 =$     alcohol content (% mas) of the mixture of a second content (initial)

$x_3 =$     alcohol content (% mas) of the mixture of a third content (final)

$C_{pr} =$     partial remaining contraction (normalised, if $Vf_{mél\,3} = 100$)

8. Device for carrying out of the process according to one of the claims 1 to 7, **characterized in that** it comprises the following elements:

- at least two containers for receiving the staring liquids containing the alcohol-water mixtures,

- a mixing container for the reception of the liquids to mix for the preparation of the mixture, optionally comprising an agitating means,

- a system of tubing and valves which are connected with the reception containers for the starting liquids and with the mixing container, such that the required quantities of the contained liquids can be led in the mixing container by controlling the valves,

- a computer system for controlling of the valves, whereby the computer system is programmed for controlling the valves, based on a table containing a plurality of values, which are entered into the computer, as % vol and the appropriate density in the range up to 100 % at constant temperature, or based on a mathematical function, which describes the alcohol content as function of the density at constant temperature, thus the correct volumes are led into the mixing container to receive a mixture with the exactly desired volume and alcohol content.

Fig. 1

Fig. 2

**Fig. 3**

Fig. 4

Fig. 5

EP 1 112 992 B1

Fig. 6

EP 1 112 992 B1

Fig. 7